# EUROPEAN PATENT APPLICATION

(11) **EP 3 904 968 A1**
(43) Date of publication of application: **03.11.2021**
(21) Application number: 19905568.2
(22) Date of filing: 25.12.2019
(51) Int. Cl.: G04G 13/02, A61B 5/11, A61B 5/113, A61B 5/16

(54) **INFORMATION PROCESSING DEVICE, INFORMATION PROCESSING METHOD, INFORMATION PROCESSING PROGRAM, AND INFORMATION PROCESSING SYSTEM**

(30) Priority: 28.12.2018 JP 2018248118
(71) Applicant: Minami, Noriyuki, Kyoto-shi, Kyoto 605-0983 (JP)
(72) Inventor: HATTORI, Yurie, Kyoto-shi Kyoto 605-0983 (JP); KAWAMURA, Kazuhiro, Kyoto-shi Kyoto 605-0983 (JP); YAMAMOTO, Yuriko, Kyoto-shi Kyoto 605-0983 (JP); HATTORI, Sadayoshi, Kyoto-shi Kyoto 605-0983 (JP); UOSAWA, Junki, Kyoto-shi Kyoto 605-0983 (JP); KONDO, Masahiro, Kyoto-shi Kyoto 605-0983 (JP); SAITO, Koji, Kyoto-shi Kyoto 605-0983 (JP)
(74) Representative: Bardehle Pagenberg Partnerschaft mbB Patentanwälte Rechtsanwälte
(86) International application number: PCT/IB2019/061350
(87) International publication number: WO 2020/136589

(57) **Abstract**

An information processing apparatus according to one aspect includes a time counter that manages time, a notification unit that performs a notification operation for waking a user up, a sensing unit that wirelessly senses a signal in accordance with motion of the user, and a controller that controls the notification unit to perform the notification operation when a notification condition is satisfied and controls the notification unit to stop the notification operation when a notification stop condition is satisfied based on a result of sensing by the sensing unit.

## Description

### TECHNICAL FIELD

The present disclosure relates to an information processing apparatus that manages sleep of a user.

### BACKGROUND ART

An apparatus that determines a sleep state of a user based on information on motion of a body caused by breathing by a user has conventionally been proposed (see PTL 1).

### CITATION LIST

### PATENT LITERATURE

PTL 1: Japanese Patent Laying-Open No. 2014-14708

### SUMMARY OF INVENTION

### TECHNICAL PROBLEM

The conventional apparatus simply determines the sleep state of the user and starts performing prescribed processing based on a result of determination, and it has not managed sleep and getting-up of the user as a whole.

The present disclosure was made to solve a problem as above, and an object of the present disclosure is to provide an information processing apparatus, an information processing method, an information processing program, and an information processing system capable of managing sleep and getting-up of a user as a whole.

### SOLUTION TO PROBLEM

An information processing apparatus according to one aspect includes a time counter that manages time, a notification unit that performs a notification operation for waking a user up, a sensing unit that wirelessly senses a signal in accordance with motion of the user, and a controller that controls the notification unit to perform the notification operation when a notification condition is satisfied and controls the notification unit to stop the notification operation when a notification stop condition is satisfied based on a result of sensing by the sensing unit.

The controller controls the notification unit to perform the notification operation when the notification condition is satisfied, and controls the notification unit to stop the notification operation when the notification stop condition is satisfied based on the result of sensing by the sensing unit. Therefore, sleep and getting-up of the user as a whole can be managed.

Preferably, the sensing unit includes a Doppler sensor. The controller includes a body motion sensing unit that senses body motion of the user based on an output from the Doppler sensor, a sleep state estimator that estimates a state of sleep of the user based on the output from the Doppler sensor, and a notification controller that controls the notification unit to perform the notification operation when the notification condition is satisfied and controls the notification unit to stop the notification operation when the notification stop condition is satisfied based on the result of sensing by the body motion sensing unit.

When body motion of the user is sensed and the notification stop condition is satisfied based on the result of sensing, the notification controller controls the notification unit to stop the notification operation. Therefore, the notification operation can readily be stopped without giving an instruction to the apparatus.

Preferably, the sleep state estimator estimates the state of sleep of the user by sensing a breath signal from the Doppler sensor.

Since the sleep state estimator estimates the sleep state of the user, sleep of the user can be managed.

Preferably, the body motion sensing unit provides a detection value indicating magnitude of body motion of the user. The controller further includes an accumulator that calculates a cumulative value from the detection value. The notification controller controls the notification unit to stop the notification operation when the cumulative value reaches a threshold value at which the notification stop condition is satisfied.

When the cumulative value of detection values representing magnitude of body motion of the user reaches the threshold value, the notification controller controls the notification unit to stop the notification operation. Therefore, a prescribed amount of body motion of the user is stimulated, and hence the user can be prompted to get up.

Preferably, the accumulator may calculate the cumulative value from detection values over a period of a predetermined length.

The accumulator calculates the cumulative value from detection values over the prescribed period. Therefore, since a prescribed amount of body motion of the user over a prescribed period can be stimulated, the user can be prompted to get up.

Preferably, the accumulator may calculate the cumulative value from detection values over a recent detection period.

The accumulator calculates the cumulative value from detection values over the recent detection period. Therefore, since a prescribed amount of body motion of the user over the recent detection period can be stimulated, the user can be prompted to get up.

Preferably, the controller may further include a representation controller that controls a display to show information indicating the cumulative value.

Since the representation controller controls the display to show information indicating the cumulative value, body motion of the user can be stimulated and the user can be prompted to get up.

Preferably, the representation controller may control the display to show an indicator varied in length in accordance with magnitude of the cumulative value toward a position indicating the threshold value.

Since the length of the indicator is varied in accordance with magnitude of the cumulative value, body motion of the user can be stimulated and the user can be prompted to get up.

Preferably, when a result of sensing by the body motion sensing unit indicates continued body motion of the user for a predetermined period, the notification controller may control the notification unit to stop the notification operation.

When the result of sensing by the body motion sensing unit indicates continued body motion of the user for a predetermined period, the notification controller controls the notification unit to stop the notification operation. Therefore, continued body motion of the user can be stimulated and the user can be prompted to get up.

Preferably, when the result of sensing by the body motion sensing unit does not indicate detection of body motion of the user while the state of sleep of the user estimated by the sleep state estimator falls under wake, the notification controller may control the notification unit not to stop the notification operation.

When body motion is not detected even though the state of sleep of the user falls under wake, the notification operation is not stopped. Therefore, the user can be prompted to get up.

An information processing method according to one aspect includes managing time, performing a notification operation for waking a user up when a notification condition is satisfied, wirelessly sensing a signal in accordance with motion of the user, and stopping the notification operation when a notification stop condition is satisfied based on a result of sensing.

The notification operation is performed when the notification condition is satisfied, and the notification operation is stopped when the notification stop condition is satisfied based on the result of sensing. Therefore, sleep and getting-up of the user as a whole can be managed.

An information processing program according to one aspect executed by a computer including a notification unit that performs a notification operation for waking a user up and a sensing unit that wirelessly senses a signal in accordance with motion of the user causes the computer to perform managing time, controlling the notification unit to perform the notification operation when a notification condition is satisfied, and controlling the notification unit to stop the notification operation when a notification stop condition is satisfied based on a result of sensing by the sensing unit.

The notification operation is performed when the notification condition is satisfied, and the notification operation is stopped when the notification stop condition is satisfied based on the result of sensing. Therefore, sleep and getting-up of the user as a whole can be managed.

An information processing system according to one aspect includes a time counter that manages time, a notification unit that performs a notification operation for waking a user up, a sensing unit that wirelessly senses a signal in accordance with motion of the user, and a controller that controls the notification unit to perform the notification operation when a notification condition is satisfied and controls the notification unit to stop the notification operation when a notification stop condition is satisfied based on a result of sensing by the sensing unit.

The notification unit is controlled to perform the notification operation when the notification condition is satisfied, and the notification unit is controlled to stop the notification operation when the notification stop condition is satisfied based on the result of sensing by the sensing unit. Therefore, sleep and getting-up of the user as a whole can be managed.

### ADVANTAGEOUS EFFECTS OF INVENTION

According to the above, the information processing apparatus, the information processing method, and the information processing program in the present disclosure can manage sleep and getting-up of the user as a whole.

### BRIEF DESCRIPTION OF DRAWINGS

Fig. 1 is a schematic block diagram of a configuration of a sleep management system 1 based on an embodiment.
Fig. 2 is a schematic block diagram of a configuration of a sleep alarm apparatus 2 based on the embodiment.
Fig. 3 is a schematic block diagram of a configuration of a server 6 based on the embodiment.
Fig. 4 is a schematic block diagram of a configuration of a terminal 8 based on the embodiment.
Fig. 5 is a conceptual diagram illustrating a form of use of sleep alarm apparatus 2 based on the embodiment.
Fig. 6 is a diagram illustrating an alarm setting screen set in terminal 8 based on the embodiment.
Fig. 7 is a diagram illustrating a get-up mode based on the embodiment.
Fig. 8 is a diagram illustrating alarm issuance (notification operation) stop processing based on the embodiment.
Fig. 9 is a diagram illustrating a functional block diagram of sleep alarm apparatus 2 based on the embodiment.
Fig. 10 is a diagram illustrating a flow of processing for an alarm function based on the embodiment.
Fig. 11 is a diagram illustrating a flow of absence rechecking processing based on the embodiment.
Fig. 12 is a diagram illustrating a flow of alarm issuance (notification operation) start processing based on the embodiment.
Fig. 13 is a diagram illustrating a flow of alarm issuance (notification operation) stop processing based on the embodiment.
Fig. 14 is a diagram illustrating a flow of stop determination processing based on the embodiment.
Fig. 15 is a diagram illustrating bar representation on a display 21 based on the embodiment.
Fig. 16 is a diagram illustrating a flow of alarm function quitting processing based on the embodiment.
Fig. 17 is a diagram illustrating a flow of processing for a back-to-sleep snooze function based on the embodiment.
Fig. 18 is a diagram illustrating a flow of processing for a reliable alarm function based on the embodiment.
Fig. 19 is a conceptual diagram illustrating a light-sleep induction function.
Fig. 20 is a diagram illustrating a flow of processing for the light-sleep induction function based on the embodiment.
Fig. 21 is a diagram illustrating a flow of light-sleep induction function start processing based on the embodiment.
Fig. 22 is a diagram illustrating a flow of processing for a morning word function based on the embodiment.
Fig. 23 is a diagram illustrating a flow of morning word function start processing based on the embodiment.
Fig. 24 is a diagram illustrating a top screen 500 of terminal 8 based on the embodiment.
Fig. 25 is a diagram illustrating a flow of sleep information checking processing in terminal 8 based on the embodiment.
Fig. 26 is a diagram illustrating a sleep detail screen 1100 (No. 1) based on the embodiment.
Fig. 27 is a diagram illustrating sleep detail screen 1100 (No. 2) based on the embodiment.
Fig. 28 is a diagram illustrating sleep detail screen 1100 (No. 3) based on the embodiment.
Fig. 29 is a diagram illustrating sleep detail screen 1100 (No. 4) based on the embodiment.
Fig. 30 is a diagram illustrating a sleep data list screen 1000 based on the embodiment.

### DESCRIPTION OF EMBODIMENTS

This embodiment will be described in detail with reference to the drawings. The same or corresponding elements in the drawings have the same reference characters allotted and description thereof will not be repeated.

An information processing apparatus in the present embodiment will be described as a sleep alarm apparatus by way of example. A portable (also referred to as mobile) apparatus or a stationary apparatus may be applicable.

### <Configuration of Sleep Management System>

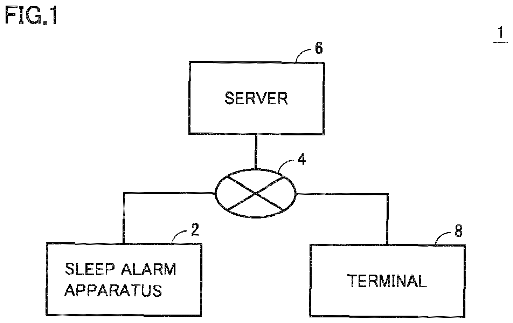

Fig. 1 is a schematic block diagram of a configuration of a sleep management system 1 based on an embodiment.

As shown in Fig. 1, sleep management system 1 includes a sleep alarm apparatus 2, a server 6, and a terminal 8 that are connected to one another over a network 4.

Information can be transmitted and received among sleep alarm apparatus 2, server 6, and terminal 8 over network 4. Network 4 may adopt any of wireless communication and wired communication.

Terminal 8 may be a portable (also referred to as mobile) apparatus such as a portable telephone or a smartphone or a stationary apparatus such as a personal computer.

Sleep alarm apparatus 2 is an apparatus that manages sleep and getting-up of a user as a whole.

Sleep alarm apparatus 2 performs an alarm function for waking a user up and performs a sensor function to wirelessly sense a signal in accordance with motion of the user. Sleep alarm apparatus 2 performs a notification operation by output of alarm sound from a speaker (notification unit) when a notification condition is satisfied, and stops output of alarm sound from the speaker (notification unit) when a notification stop condition is satisfied based on a result of sensing by the sensor function.

Sleep alarm apparatus 2 also performs a sleep analysis function to analyze sleep data.

Terminal 8 can set the alarm function of sleep alarm apparatus 2 and can obtain a sleep state of the user from sleep alarm apparatus 2 or server 6 and show the sleep state.

Sleep data obtained in sleep alarm apparatus 2 is stored in server 6.

### <Configuration of Sleep Alarm Apparatus 2>

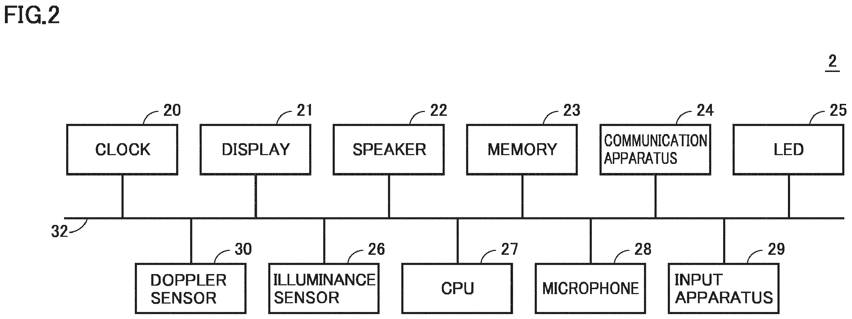

Fig. 2 is a schematic block diagram of a configuration of sleep alarm apparatus 2 based on the embodiment.

As shown in Fig. 2, sleep alarm apparatus 2 includes a clock 20, a display 21, a speaker 22, a memory 23, a communication apparatus 24, an LED 25, an illuminance sensor 26, a CPU 27, a microphone 28, an input apparatus 29, a Doppler sensor 30, and an internal bus 32. Components are connected through internal bus 32.

CPU 27 is an information processing unit that performs various types of information processing performed in sleep alarm apparatus 2. CPU 27 performs various types of information processing by using memory 23. Various programs to be executed in sleep alarm apparatus 2 and data relating to sleep measured in real time when the user sleeps are stored in memory 23.

Though memory 23 is described as a storage contained in sleep alarm apparatus 2, for example, a storage medium attachable to and removable from sleep alarm apparatus 2 such as an optical disc or a cartridge may be applicable or both of the storage and the storage medium as such may be applicable.

CPU 27 implements various functional blocks based on a program stored in memory 23.

Clock 20 performs a function to count time.

Display 21 shows information such as time.

Speaker 22 provides alarm sound as notification sound.

Communication apparatus 24 is an interface for communication with an external apparatus (server 6 and terminal 8) over network 4.

LED 25 is turned on in response to an instruction and lights up an area around sleep alarm apparatus 2.

Microphone 28 accepts external audio input.

Input apparatus 29 includes various operation buttons.

Doppler sensor 30 emits radio waves (microwaves) to an object and wirelessly senses a signal (reflected waves) in accordance with motion of the object (user).

In sleep alarm apparatus 2, the sleep state of the user can be determined as any of four types, for example, of "deep sleep," "light sleep," "REM sleep," and "wake" based on information obtained by detection of reflected waves converted from microwaves emitted from Doppler sensor 30 due to slight motion caused by breathing by the user. A known technique as described, for example, in PTL 1 described previously can be used as a specific method of determination processing. In determining the sleep state, information on slight motion caused by heartbeats may also be taken into account.

Probability as to whether or not a living body is present within an area of monitoring by Doppler sensor 30 can be calculated based on detection of motion caused by breathing described above. For example, whether or not a user is within a monitoring region on the bed can thus be determined.

Sleep alarm apparatus 2 can also detect relatively large body motion such as turn-over or an operation to wave a hand based on a known technique using Doppler sensor 30,. Distinction from slight motion due to breath or heartbeat described above can be made, for example, based on an amount of change between emitted waves and reflected waves or periodicity.

Relatively large motion of the user such as turn-over or an operation to wave a hand may be called "body motion," and such motion, together with slight motion such as breath or heartbeat, may be called "motion."

### <Configuration of Server 6>

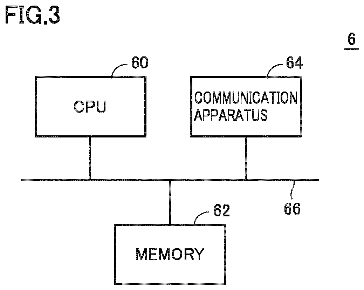

Fig. 3 is a schematic block diagram of a configuration of server 6 based on the embodiment.

As shown in Fig. 3, server 6 includes a CPU 60, a memory 62, a communication apparatus 64, and an internal bus 66. Components are connected through internal bus 66.

CPU 60 is an information processing unit that performs various types of information processing performed in server 6. CPU 60 performs various types of information processing by using memory 62.

Though memory 62 is described as a storage contained in server 6, for example, a storage medium attachable to and removable from server 6 such as an optical disc or a cartridge may be applicable or both of the storage and the storage medium as such may be applicable.

Communication apparatus 64 is an interface for communication with an external apparatus (sleep alarm apparatus 2 and terminal 8) over network 4.

### <Configuration of Terminal 8>

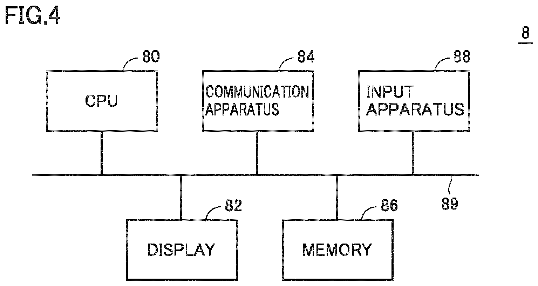

Fig. 4 is a schematic block diagram of a configuration of terminal 8 based on the embodiment.

As shown in Fig. 4, terminal 8 includes a CPU 80, a display 82, a communication apparatus 84, a memory 86, an input apparatus 88, and an internal bus 89. Components are connected through internal bus 89.

CPU 80 is an information processing unit that performs various types of information processing performed in terminal 8. CPU 80 performs various types of information processing by using memory 86.

### Input apparatus 88 includes a touch panel.

Communication apparatus 84 is an interface for communication with an external apparatus (sleep alarm apparatus 2 and server 6) over network 4.

Though memory 86 is described as a storage contained in terminal 8, for example, a storage medium attachable to and removable from terminal 8 such as an optical disc or a cartridge may be applicable or both of the storage and the storage medium as such may be applicable.

### <Form of Use of Sleep Alarm Apparatus 2>

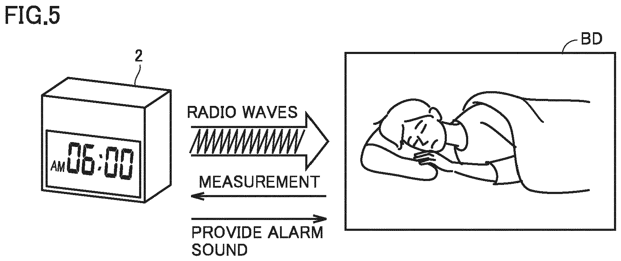

Fig. 5 is a conceptual diagram illustrating a form of use of sleep alarm apparatus 2 based on the embodiment.

As shown in Fig. 5, sleep alarm apparatus 2 is arranged adjacently to a bed BD or the like of a user.

Sleep alarm apparatus 2 measures reflected waves of radio waves emitted from Doppler sensor 30 to a user and monitors the sleep state of the user based on the reflected waves. A region of monitoring by sleep alarm apparatus 2 corresponds to a prescribed region (prescribed area) where the user sleeps on bed BD.

Sleep alarm apparatus 2 performs an alarm function. In the present example, sleep alarm apparatus 2 provides alarm sound from speaker 22 when a notification condition is satisfied. Display 21 shows "AM 6:00" as the current time counted by clock 20, by way of example.

### <Alarm Setting Screen>

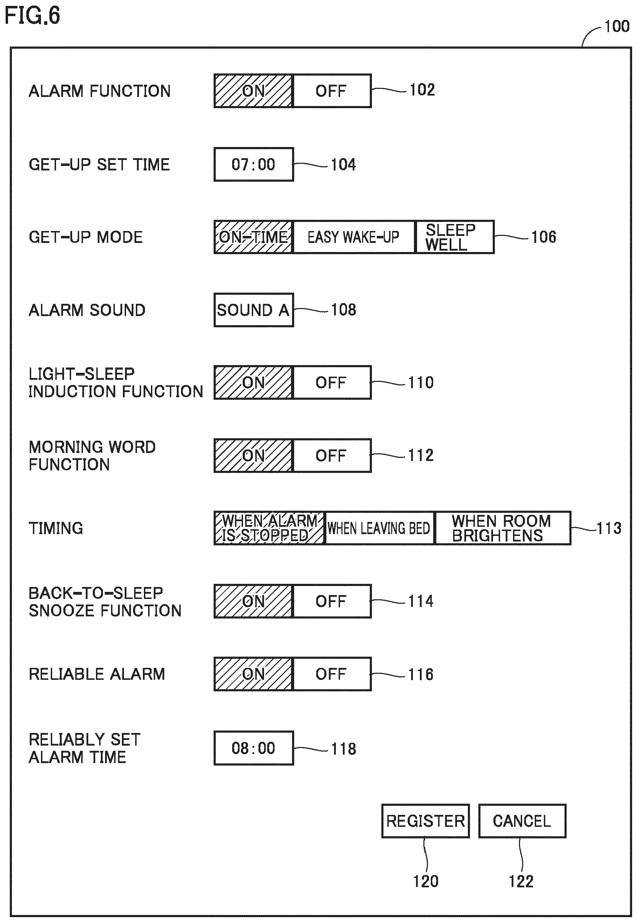

Fig. 6 is a diagram illustrating an alarm setting screen set in terminal 8 based on the embodiment.

Fig. 6 shows an alarm setting screen 100 shown on display 82 of terminal 8. Display 82 of terminal 8 is provided with a touch panel as input apparatus 88, and processing for setting the alarm function of sleep alarm apparatus 2 can be performed through the touch panel. Without being limited to the touch panel, setting processing can also be performed by using a keyboard or the like.

Alarm setting screen 100 is provided with an alarm function on-and-off switch button 102, a get-up set time input field 104, a get-up mode switch button 106, an alarm sound selection button 108, a light-sleep induction function on-and-off switch button 110, a morning word function on-and-off switch button 112, a timing selection button 113, a back-to-sleep snooze function on-and-off switch button 114, a reliable alarm function on-and-off switch button 116, a reliable alarm time input field 118, a register button 120, and a cancel button 122.

Alarm function on-and-off switch button 102 is a button for switching on or off the alarm function. When the alarm function is on, the notification operation is performed. When the alarm function is off, the prescribed notification operation is not performed. In the present example, an example in which the alarm function is set to "on" is shown. When the alarm function is set to "off", each switch button or selection button or the like is set to an inactive state.

Get-up set time input field 104 is a field where the user enters get-up set time. In the present example, an example in which "07:00" is set in get-up set time input field 104 is shown.

Get-up mode switch button 106 is a button to switch among get-up modes. In the present example, a plurality of get-up modes are provided. Specifically, three types of get-up modes of an "on-time" mode, an "easy wake-up" mode, and a "sleep well" mode are provided.

The "on-time" mode refers to a mode set when a user wants to get up at the time that the user set in get-up set time input field 104.

The "easy wake-up" mode refers to a mode set when the user wants to get up easily at the time that the user set in get-up set time input field 104.

The "sleep well" mode refers to a mode set when the user wants to soundly sleep. When the "sleep well" mode is set, the notification operation in accordance with time set in get-up set time input field 104 is not performed. In the present example, an example in which the "on-time" mode is set is shown.

Alarm sound selection button 108 is a button for selecting alarm sound in performing the notification operation. By selecting the button, alarm sound can be selected from among a plurality of types, although not shown. In the present example, an example in which selection of sound A is made is shown.

Light-sleep induction function on-and-off switch button 110 is a button for setting on or off of the light-sleep induction function. When the light-sleep induction function is on, prescribed processing for inducing light sleep is performed. When the light-sleep induction function is off, the prescribed processing is not performed. In the present example, an example in which the light-sleep induction function is set to "on" is shown.

Morning word function on-and-off switch button 112 is a button for setting on or off of the morning word function. When the morning word function is on, prescribed processing for giving a morning word is performed. When the morning word function is off, the prescribed processing is not performed. In the present example, an example in which the morning word function is set to "on" is shown.

Timing selection button 113 is a button for selecting timing to perform the morning word function. In the present example, an example in which selection from among "when alarm is stopped," "when leaving bed," and "when room brightens" can be made is shown. When "when alarm is stopped" is selected, the morning word function is performed when alarm is stopped. When "when leaving bed" is selected, the morning word function is performed when the user leaves the bed. When "when room brightens" is selected, the morning word function is performed when the room brightens. In the present example, an example in which timing to perform the morning word function is set to "when alarm is stopped" is shown.

Back-to-sleep snooze function on-and-off switch button 114 is a button for setting on or off of the back-to-sleep snooze function. When the back-to-sleep snooze function is on, prescribed processing for the back-to-sleep snooze function is performed. When the back-to-sleep snooze function is off, the prescribed processing is not performed. In the present example, an example in which the back-to-sleep snooze function is set to "on" is shown.

Reliable alarm function on-and-off switch button 116 is a button for setting on or off of the reliable alarm function. When the reliable alarm function is on, prescribed processing for the reliable alarm function is performed. When the reliable alarm function is off, the prescribed processing is not performed. In the present example, an example in which the reliable alarm function is set to "on" is shown.

Reliable alarm time input field 118 is a field where the user enters time to give reliable alarm. In the present example, an example in which "08:00" is set in reliable alarm time input field 118 is shown.

Register button 120 is a button for performing registration processing for registering in sleep alarm apparatus 2, setting information set in alarm setting screen 100.

Cancel button 122 is a button for canceling registration processing for registering in sleep alarm apparatus 2, setting information set in alarm setting screen 100.

When register button 120 in alarm setting screen 100 shown on display 82 is selected, terminal 8 transmits various types of setting information set in alarm setting screen 100 (which is also simply referred to as "setting information" below) to sleep alarm apparatus 2 over network 4.

Sleep alarm apparatus 2 receives setting information transmitted from terminal 8 and has the setting information stored in memory 23. Sleep alarm apparatus 2 performs the alarm function based on the setting information stored in memory 23.

When cancel button 122 in alarm setting screen 100 shown on display 82 is selected, terminal 8 quits showing alarm setting screen 100 and quits processing for registering the setting information.

When alarm function on-and-off switch button 102 in alarm setting screen 100 is set to "off" and register button 120 is selected, the alarm function set in advance in sleep alarm apparatus 2 is set to off.

Though an example in which processing for setting the alarm function is performed in terminal 8 is described in the present example, without being limited as such, similar setting processing can also be performed by using input apparatus 29 of sleep alarm apparatus 2.

### <Get-Up Mode>

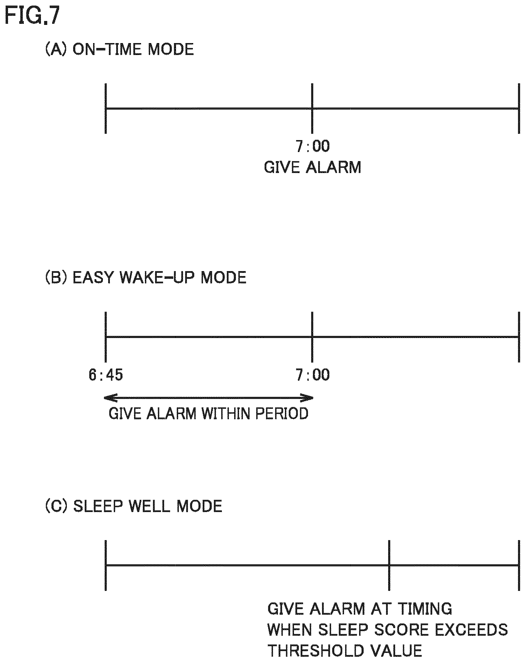

Fig. 7 is a diagram illustrating the get-up mode based on the embodiment.

Fig. 7 (A) shows an exemplary on-time mode.

In the "on-time" mode, alarm is given (the notification operation is performed) at get-up set time set in get-up set time input field 104.

Fig. 7 (B) shows an exemplary "easy wake-up" mode.

In the "easy wake-up" mode, alarm is given (the notification operation is performed) between timing fifteen minutes before the get-up set time set in get-up set time input field 104 and the get-up set time. Specifically, turn-over of the user is detected during this period and alarm is given (the notification operation is performed) in response to detection. When the user turns over, alarm is given (the notification operation is performed) at the timing of sensing of turn-over so that the user can comfortably wake up.

Fig. 7 (C) shows an exemplary "sleep well" mode.

In the "sleep well" mode, alarm is given (the notification operation is performed) after the user sleeps soundly regardless of time set in get-up set time input field 104. Though description will be given later, specifically, a sleep score indicating a sleep sufficiency degree of the user is calculated, and when the sleep score exceeds a threshold value, the user is determined as having slept soundly and alarm is given (the notification operation is performed). In other words, since alarm is not given (the notification operation is not performed) until the sleep score exceeds the threshold value, the user can reliably take a rest by sleeping. When the sleep score exceeds the threshold value, alarm is given (the notification operation is performed) and hence the user can be prevented from taking unnecessary sleep.

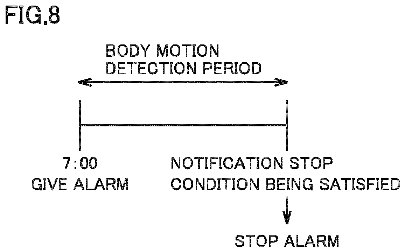

Fig. 8 is a diagram illustrating alarm issuance (notification operation) stop processing based on the embodiment.

As shown in Fig. 8, in the present example, alarm is given (the notification operation is performed) when the notification condition is satisfied, and issuance of alarm ends (the notification operation is stopped) when the notification stop condition is satisfied.

Specifically, when alarm is given (the notification operation is performed), sleep alarm apparatus 2 detects body motion of the user (a body motion detection period). Sleep alarm apparatus 2 determines whether or not the notification stop condition is satisfied based on a result of detection during the body motion detection period, and when the notification stop condition is satisfied, alarm issuance ends (the notification operation is stopped).

By way of example of the notification stop condition, in the present example, whether or not there has been a prescribed amount or more of motion of the user during the body motion detection period is determined. When the prescribed amount or more of motion of the user is detected, the notification stop condition is determined as being satisfied and alarm issuance ends (the notification operation is stopped).

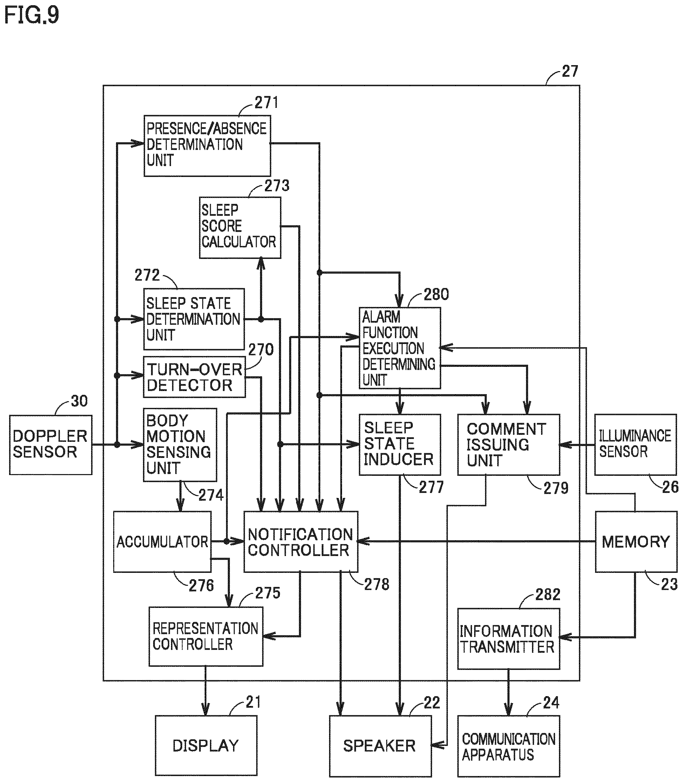

Fig. 9 is a diagram illustrating a functional block diagram of sleep alarm apparatus 2 based on the embodiment.

Referring to Fig. 9, CPU 27 of sleep alarm apparatus 2 implements various functional blocks.

Specifically, CPU 27 implements a plurality of functional blocks based on a program stored in memory 23. In the present example, CPU 27 includes a turn-over sensing unit 270, a presence/absence determination unit 271, a sleep state determination unit 272, a sleep score calculator 273, a body motion sensing unit 274, a representation controller 275, an accumulator 276, a sleep state inducer 277, a notification controller 278, a comment issuing unit 279, an alarm function execution determining unit 280, and an information transmitter 282.

Alarm function execution determining unit 280 determines whether or not to perform the alarm function of sleep alarm apparatus 2. When alarm function execution determining unit 280 determines to perform the alarm function of sleep alarm apparatus 2, it instructs notification controller 278 to perform an alarm issuance start operation.

Presence/absence determination unit 271 determines whether a user is present or absent (not present) in a prescribed region (prescribed area) corresponding to a monitoring region based on a detection signal from Doppler sensor 30. In the present example, whether the user is present or absent (not present) on the bed is determined.

Sleep state determination unit 272 determines in real time, the sleep state of the user based on a detection signal from Doppler sensor 30. Specifically, sleep state determination unit 272 determines the sleep state of the user as a deep sleep state, a light sleep state, a REM sleep state, or a wake state, based on a detection signal from Doppler sensor 30.

Sleep score calculator 273 calculates the sleep score that represents the sleep sufficiency degree based on a result of determination of the sleep state made in sleep state determination unit 272. Sleep score calculator 273 provides the calculated sleep score to notification controller 278.

Body motion sensing unit 274 calculates an amount of body motion of the user based on a detection signal from Doppler sensor 30.

Accumulator 276 provides a cumulative value obtained by accumulation of amounts of body motion of the user over a prescribed period that is calculated by body motion sensing unit 274.

Notification controller 278 controls issuance of alarm (the notification operation) through speaker 22. Specifically, by way of example, notification controller 278 obtains get-up set time in the setting information stored in memory 23, and when the notification condition is satisfied, it controls speaker 22 to give alarm (perform the notification operation) at the get-up set time. When the notification stop condition is satisfied, notification controller 278 controls speaker 22 to quit issuance of alarm (stop the notification operation).

Representation controller 275 controls representation on display 21. Though description will be given later, representation controller 275 has an indicator corresponding to a cumulative value provided from accumulator 276 shown.

When the notification stop condition is satisfied, notification controller 278 notifies representation controller 275 of that fact, and representation controller 275 has representation of the indicator end in response to the notification.

Sleep state inducer 277 checks the sleep state of the user, and when the sleep state of the user falls under deep sleep, it induces the sleep state to light sleep.

Comment issuing unit 279 has various types of information given through speaker 22. For example, the comment issuing unit has necessary information such as weather or a temperature given.

Turn-over sensing unit 270 determines whether or not the user has turned over based on a detection signal from Doppler sensor 30. When turn-over sensing unit 270 determines that the user has turned over, it provides a sensing signal to notification controller 278.

Information transmitter 282 transmits to server 6 as sleep data, the setting information stored in memory 23 and various types of information relating to sleep based on a detection signal from Doppler sensor 30. Server 6 receives sleep data through communication apparatus 64 and has the sleep data stored in memory 62. Processing for checking the sleep state which will be described later is performed based on the sleep data stored in memory 62.

### <Alarm Function>

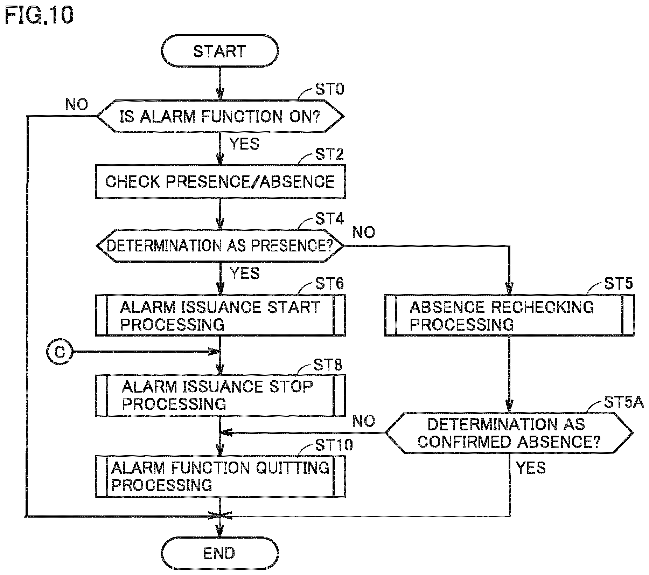

Fig. 10 is a diagram illustrating a flow of processing for the alarm function based on the embodiment.

As shown in Fig. 10, CPU 27 determines whether or not the alarm function is ON (step ST0). Alarm function execution determining unit 280 determines whether or not the setting information includes information indicating that the alarm function is ON.

When CPU 27 determines in step ST0 that the alarm function is ON (YES in step ST0), it checks presence/absence (step ST2). Specifically, presence/absence determination unit 271 determines presence/absence of the user in a prescribed region based on a detection signal from Doppler sensor 30, and provides a result of determination to alarm function execution determining unit 280.

CPU 27 makes determination as to presence (step ST4). Alarm function execution determining unit 280 makes determination as to presence based on the result of determination as presence/absence from presence/absence determination unit 271.

When CPU 27 makes determination as presence (YES in step ST4), it performs alarm issuance start processing (step ST6). When alarm function execution determining unit 280 determines that the result of determination from presence/absence determination unit 271 indicates presence, it instructs notification controller 278 to perform the alarm issuance start processing.

Then, CPU 27 performs alarm issuance stop processing (step ST8). Details of the alarm issuance stop processing will be described later.

Then, CPU 27 performs alarm function quitting processing (step ST10). Details of the alarm function quitting processing will be described later.

Then, the process ends (end).

When CPU 27 makes determination not as presence in step ST2 (NO in step ST4), it performs absence rechecking processing (step ST5).

Details of the absence rechecking processing will be described later.

CPU 27 determines whether or not a result of the absence rechecking processing indicates confirmed absence (step ST5A).

When CPU 27 determines absence as having been confirmed (YES in step ST5A), the process ends (end). The process ends without alarm function execution determining unit 280 giving an instruction to notification controller 278.

On the other hand, when CPU 27 determines absence as not having been confirmed (NO in step ST5A), the process proceeds to step ST10 and performs the alarm function quitting processing. Alarm function execution determining unit 280 instructs notification controller 278 to perform the alarm function quitting processing (end). When the result of determination as to presence/absence from presence/absence determination unit 271 indicates that absence has been confirmed, alarm function execution determining unit 280 does not instruct notification controller 278 to perform the alarm issuance start processing. Therefore, alarm is not given (the notification operation is not performed).

When CPU 27 determines in step ST0 that the alarm function is not ON (NO in step ST0), the process ends (end). When the setting information includes information that the alarm function is OFF, alarm function execution determining unit 280 does not instruct notification controller 278 to perform the alarm issuance start processing. Therefore, alarm is not given (the notification operation is not performed).

Through the processing, when the user desires to turn off the alarm function (OFF of the alarm function), the alarm function is not performed. Therefore, useless alarm issuance (the notification operation) can be suppressed and user's intention can be reflected.

### <Absence Rechecking Processing>

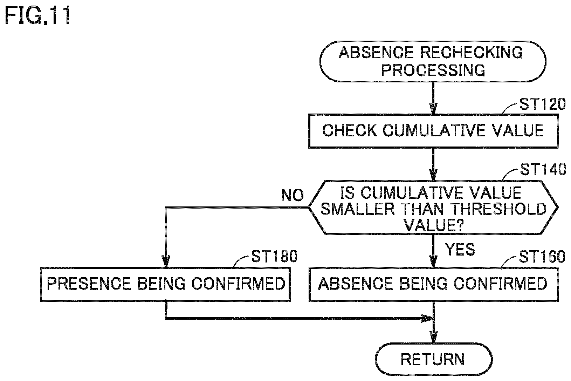

Fig. 11 is a diagram illustrating a flow of absence rechecking processing based on the embodiment.

As shown in Fig. 11, CPU 27 checks a cumulative value (step ST120). Alarm function execution determining unit 280 checks the cumulative value which is accumulation of the amounts of body motion of the user over a prescribed period calculated by accumulator 276.

CPU 27 determines whether or not the cumulative value is smaller than a threshold value (step ST140). Alarm function execution determining unit 280 determines whether or not the cumulative value calculated by accumulator 276 is smaller than a prescribed threshold value.

When CPU 27 determines in step ST140 the cumulative value as being smaller than the threshold value (YES in step ST140), it makes determination that absence has been confirmed (step ST160). When alarm function execution determining unit 280 determines the cumulative value as being smaller than the threshold value, it makes determination that absence has been confirmed.

Then, the process ends (return).

On the other hand, when CPU 27 determines in step ST140 the cumulative value as being equal to or larger than the threshold value (NO in step ST140), it makes determination that presence has been confirmed (step ST180). When alarm function execution determining unit 280 determines the cumulative value as being equal to or larger than the threshold value, it makes determination that presence has been confirmed.

Then, the process ends (return).

Through the processing, when presence of the user is confirmed in the absence rechecking processing even when the result of determination by presence/absence determination unit 271 is incorrectly recognized as absence of the user, functional processing other than alarm issuance can be performed.

### <Alarm Issuance Start Processing>

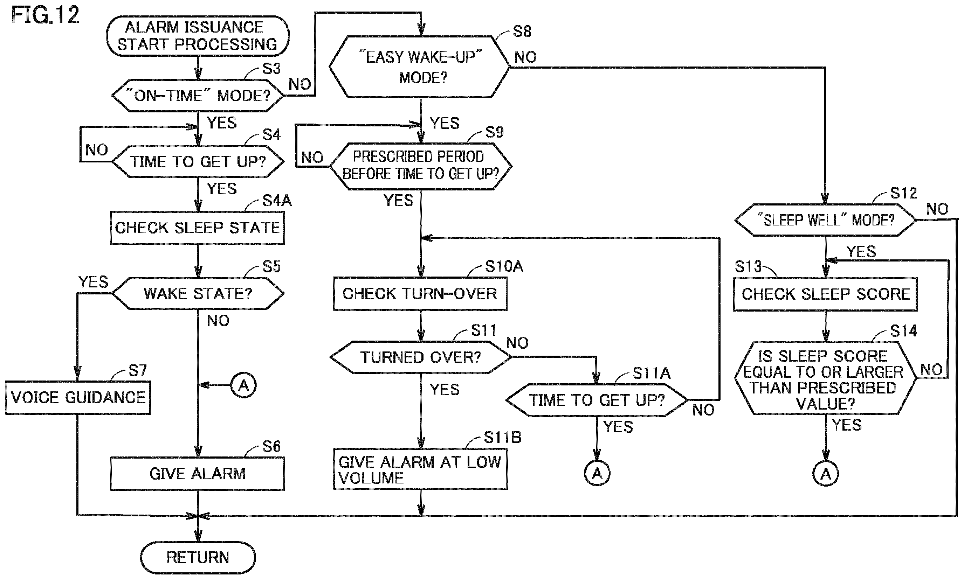

Fig. 12 is a diagram illustrating a flow of alarm issuance (notification operation) start processing based on the embodiment.

As shown in Fig. 12, CPU 27 determines whether or not the get-up mode has been set to the "on-time" mode (step S3). Notification controller 278 determines whether or not the get-up mode in the setting information stored in memory 23 has been set to the "on-time" mode.

When CPU 27 determines in step S3 that the get-up mode has been set to the "on-time" mode (YES in step S3), it determines whether or not the get-up set time has come (step S4). Specifically, notification controller 278 determines whether or not the get-up set time has come based on the get-up set time stored in memory 23.

CPU 27 maintains the state in step S4 until get-up set time comes (NO in step S4). Notification controller 278 stands by until get-up set time stored in memory 23 comes.

When CPU 27 determines that the get-up set time has come (YES in step S4), it checks the sleep state of the user (step S4A). Sleep state determination unit 272 determines the sleep state of the user based on a signal from Doppler sensor 30, and provides a result of determination to notification controller 278.

CPU 27 determines whether or not the sleep state of the user falls under the wake state (step S5). Notification controller 278 determines whether or not the sleep state falls under the wake state based on the result of determination from sleep state determination unit 272.

When CPU 27 determines that the sleep state of the user falls under the wake state (YES in step S5), it provides voice guidance (step S7). Specifically, when notification controller 278 determines that the sleep state of the user falls under the wake state, it instructs speaker 22 to provide voice guidance information. For example, guidance "Have you already waken up? If you want to turn off alarm, please turn off the alarm by moving your body." is provided as voice guidance information. Then, the process ends (return).

On the other hand, when CPU 27 determines that the sleep state of the user does not fall under the wake state (NO in step S5), it gives alarm (performs the notification operation). Notification controller 278 plays a music file of sound A included in the setting information stored in memory 23 and controls speaker 22 to provide output of the music file. Then, the process ends (return). The music file of sound A for output from speaker 22 is stored in advance in memory 23.

When CPU 27 determines in step S3 that the get-up mode has not been set to the "on-time" mode (NO in step S3), it determines whether or not the get-up mode has been set to the "easy wake-up" mode (step S8). Notification controller 278 determines whether or not the get-up mode in the setting information stored in memory 23 has been set to the "easy wake-up" mode.

When CPU 27 determines in step S8 that the get-up mode has been set to the "easy wake-up" mode (YES in step S8), it determines whether or not the current time is time a prescribed period before the get-up set time (step S9). Specifically, notification controller 278 determines whether or not the current time is time a prescribed period (fifteen minutes by way of example) before the get-up set time in the setting information.

When CPU 27 determines in step S9 that the current time is time the prescribed period before the get-up set time (YES in step S9), it checks a turn-over state (step S10A). Turn-over sensing unit 270 detects turn-over of the user based on a signal from Doppler sensor 30, and provides the result to notification controller 278.

CPU 27 determines whether or not the user has turned over (step S11). Notification controller 278 determines whether or not it has received input of a sensing signal from turn-over sensing unit 270.

When CPU 27 determines that the user has turned over (YES in step S11), it gives alarm at a low volume (performs the notification operation) (step S11B). Notification controller 278 plays a music file of sound A included in the setting information stored in memory 23 and controls speaker 22 to provide output of the music file. In this case, the music file is played at the low volume and output of the music file is provided from speaker 22. Then, the process ends (return). As a result of play and output at the low volume from speaker 22, the user can wake up easily.

On the other hand, when CPU 27 determines that the user has not turned over (NO in step S11), it determines whether or not the get-up set time has come (step S 11A). Specifically, notification controller 278 determines whether or not the get-up set time stored in memory 23 has come.

When CPU 27 determines that the get-up set time has not yet come (NO in step S11A), the process returns to step S10A and the processing above is repeated.

On the other hand, when CPU 27 determines that the get-up set time has come (YES in step S 11A), the process proceeds to "A". In other words, the process proceeds to step S6 and CPU 27 gives alarm (performs the notification operation). Notification controller 278 plays a music file of sound A included in the setting information stored in memory 23 and controls speaker 22 to provide output of the music file. Then, the process ends (return).

When CPU 27 determines in step S8 that the get-up mode has not been set to the "easy wake-up" mode (NO in step S8), it determines whether or not the get-up mode has been set to the "sleep well" mode (step S12). Specifically, notification controller 278 determines whether or not the get-up mode in the setting information stored in memory 23 has been set to the "sleep well" mode.

When CPU 27 determines in step S12 that the get-up mode has been set to the "sleep well" mode (YES in step S12), it checks the sleep score (step S13). Sleep score calculator 273 calculates the sleep score that represents the sleep sufficiency degree of the user based on a result of determination as to the sleep state by sleep state determination unit 272 and provides a result of calculation to notification controller 278. The sleep score is larger in value when the deep sleep state continues and smaller in value when the light sleep state continues. Therefore, as the number of times of awakening is larger or hours of awakening are longer, the sleep score is lower.

CPU 27 determines whether or not the sleep score is equal to or larger than a prescribed value (step S14).

Notification controller 278 determines whether or not the sleep score is equal to or larger than the prescribed value based on the result of calculation from sleep score calculator 273. The prescribed value can be set to a fixed value or can be varied depending on age or sex.

When CPU 27 determines in step S14 that the sleep score is equal to or larger than the prescribed value (YES in step S14), the process proceeds to "A". In other words, the process proceeds to step S6 and CPU 27 gives alarm (performs the notification operation). Notification controller 278 plays a music file of sound A included in the setting information stored in memory 23 and controls speaker 22 to provide output of the music file. Then, the process ends (return).

On the other hand, when CPU 27 determines in step S14 that the sleep score is lower than the prescribed value (NO in step S14), the process returns to step S13 and the processing above is repeated.

When CPU 27 determines that the get-up mode has not been set to the "sleep well" mode (NO in step S9), the process ends (return).

### <Alarm Issuance Stop Processing>

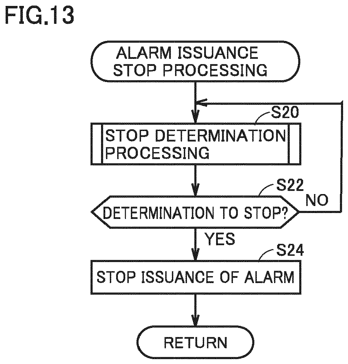

Fig. 13 is a diagram illustrating a flow of alarm issuance (notification operation) stop processing based on the embodiment.

As shown in Fig. 13, CPU 27 performs stop determination processing (step S20). Details of the stop determination processing will be described later.

Then, CPU 27 determines whether or not a result of determination in the stop determination processing indicates stop (step S22). Specifically, notification controller 278 determines whether or not the result of determination in the stop determination processing indicates stop.

Then, when CPU 27 determines that the result of determination in the stop determination processing indicates stop (YES in step S22), it stops issuance of alarm (the notification operation) (step S24). Notification controller 278 stops output of alarm sound from speaker 22. Then, the process ends (return).

On the other hand, when CPU 27 determines in step S22 that the result of determination in the stop determination processing does not indicate stop (NO in step S22), the process returns to step S20.

### <Stop Determination Processing>

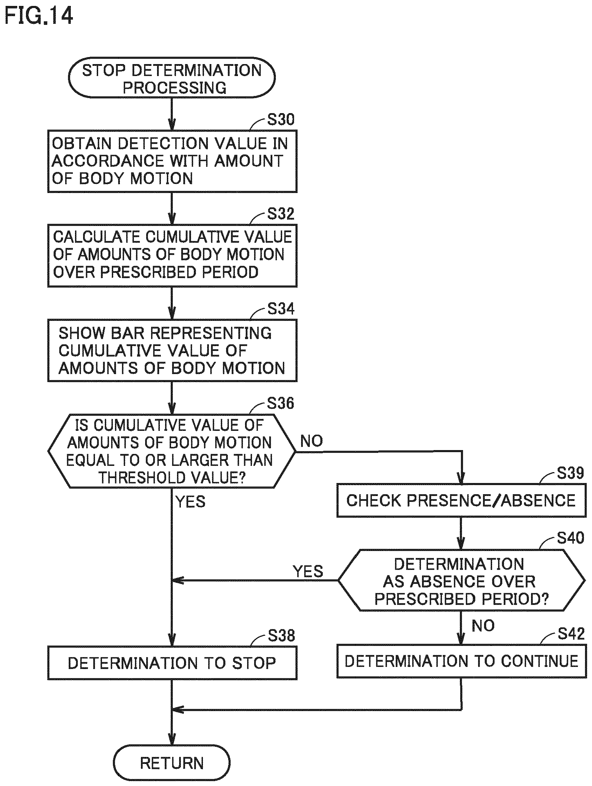

Fig. 14 is a diagram illustrating a flow of the stop determination processing based on the embodiment.

As shown in Fig. 14, CPU 27 obtains a detection value in accordance with an amount of body motion in such an operation as turn-over or waving a hand (step S30). Body motion sensing unit 274 obtains a detection value based on an output from Doppler sensor 30.

CPU 27 calculates a cumulative value of amounts of body motion over a prescribed period (step S32). Specifically, accumulator 276 calculates a cumulative value of the amounts of body motion over the prescribed period provided from body motion sensing unit 274.

CPU 27 has the cumulative value of the amounts of body motion shown in a form of a bar on display 21 (step S34). Representation controller 275 controls display 21 to show in real time a bar representing a degree of accumulation based on the cumulative value from accumulator 276.

CPU 27 determines whether or not the cumulative value of the amounts of body motion is equal to or larger than a threshold value (step S36). Notification controller 278 determines whether or not the cumulative value of the amounts of body motion provided from accumulator 276 is equal to or larger than a threshold value. Notification controller 278 obtains a threshold value of the cumulative value from memory 23.

When the cumulative value of the amounts of body motion is equal to or larger than the threshold value (YES in step S36), CPU 27 makes determination to stop (step S38). When the cumulative value of the amounts of body motion provided from accumulator 276 is equal to or larger than the threshold value, notification controller 278 makes determination to stop, as the result of determination. Then, the process ends (return).

On the other hand, when the cumulative value of the amounts of body motion is smaller than the threshold value (NO in step S36), CPU 27 checks presence/absence (step S39). Specifically, presence/absence determination unit 271 provides a result of determination as to presence/absence to notification controller 278 based on a signal from Doppler sensor 30.

CPU 27 makes determination as to absence over a specific period (step S40). Notification controller 278 determines whether or not it has obtained a result of determination as absence from presence/absence determination unit 271 over the specific period.

When CPU 27 makes determination as absence over the specific period in step S40 (YES in step S40), it makes determination to stop (step S38). When notification controller 278 makes determination as absence over the specific period, it makes determination to stop, as the result of determination. Then, the process ends (return).

On the other hand, when determination not as absence over the specific period is made in step S40 (NO in step S40), determination to continue is made (step S42). When notification controller 278 makes determination not as absence over the prescribed period, it makes determination to continue, as the result of determination. Then, the process ends (return).

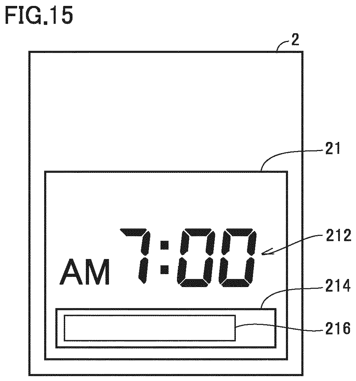

Fig. 15 is a diagram illustrating bar representation on display 21 based on the embodiment.

As shown in Fig. 15, time 212 and an indicator 214 are provided on display 21 of sleep alarm apparatus 2. Indicator 214 includes a bar 216 varied in length in correspondence with a degree of accumulation in conformity with the cumulative value. As the cumulative value over the prescribed period is larger, bar 216 is longer, and as the cumulative value over the prescribed period is smaller, bar 216 is shorter.

As the user checks bar 216, the user can move to set the cumulative value over the prescribed period to the threshold value or larger. Though the threshold value is set at the right end of indicator 214 in the present example, without being limited as such, the threshold value can be set at any position.

Any length of the prescribed period can be set, and the entire period during which alarm is given (the notification operation is performed) or a prescribed fixed recent detection period may be defined as the prescribed period.

### <Alarm Function Quitting Processing>

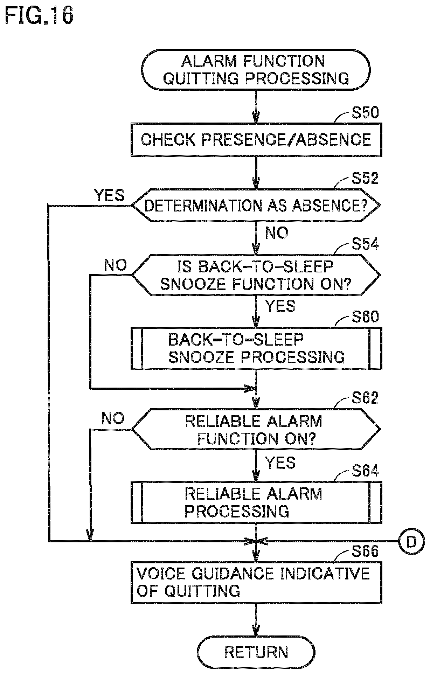

Fig. 16 is a diagram illustrating a flow of the alarm function quitting processing based on the embodiment.

As shown in Fig. 16, CPU 27 checks presence/absence (step S50). Specifically, presence/absence determination unit 271 makes determination as to presence/absence based on a signal from Doppler sensor 30 and provides a result of determination to notification controller 278.

CPU 27 makes determination as to absence (step S52). Notification controller 278 makes determination as to absence based on the result of determination as to presence/absence from presence/absence determination unit 271.

When CPU 27 makes determination as absence in step S52 (YES in step S52), it provides output of voice guidance indicative of quitting the alarm function (step S66). Notification controller 278 instructs speaker 22 to provide output of voice guidance information indicative of quitting. For example, guidance "today's alarm ends" is provided as voice guidance information indicative of quitting. Then, the process ends (return).

On the other hand, when CPU 27 determines not as absence in step S52 (NO in step S52), it determines whether or not the back-to-sleep snooze function is ON (step S54). Notification controller 278 determines whether or not the setting information includes information that the back-to-sleep snooze function is ON.

When CPU 27 determines that the back-to-sleep snooze function is ON (YES in step S54), it performs back-to-sleep snooze processing (step S60). Details of the back-to-sleep snooze processing will be described later.

When CPU 27 determines in step S54 that the back-to-sleep snooze function is not ON (NO in step S54), the process proceeds to step S62.

CPU 27 determines whether or not the reliable alarm function is ON (step S62). Notification controller 278 determines whether or not the setting information includes information that the reliable alarm function is ON.

When CPU 27 determines in step S62 that the reliable alarm function is ON, it performs reliable alarm processing (step S64). Details of the reliable alarm processing will be described later. Then, the process proceeds to step S66 and output of voice guidance indicative of quitting the alarm function is provided (step S66). Notification controller 278 instructs speaker 22 to give guidance "today's alarm ends" as voice guidance. Then, the process ends (return).

When CPU 27 determines in step S62 that the reliable alarm function is not ON, the process proceeds to step S66 and has voice guidance indicative of quitting the alarm function given (step S66). Notification controller 278 instructs speaker 22 to give guidance information indicative of quitting. For example, guidance "today's alarm ends" is given as the voice guidance information indicative of quitting. Then, the process ends (return).

### <Back-to-Sleep Snooze Function>

The back-to-sleep snooze function is a snooze function to give alarm based on detection of back-to-sleep by the user.

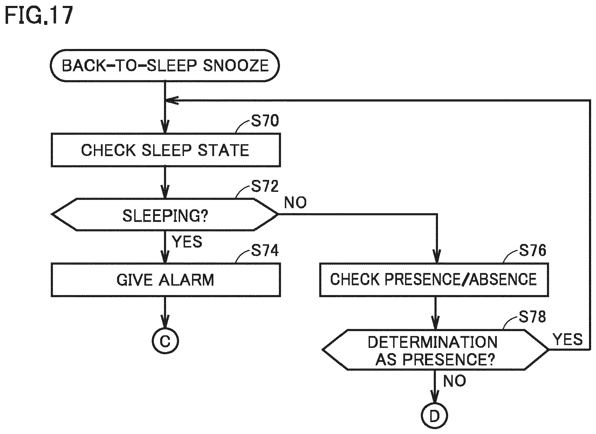

Fig. 17 is a diagram illustrating a flow of processing for the back-to-sleep snooze function based on the embodiment.

As shown in Fig. 17, CPU 27 checks the sleep state of the user (step S70). Sleep state determination unit 272 determines the sleep state of the user based on a signal from Doppler sensor 30 and provides a result of determination to notification controller 278.

CPU 27 determines whether or not the user is sleeping (step S72). Notification controller 278 determines whether or not the user is sleeping based on a result of determination from sleep state determination unit 272.

When CPU 27 determines in step S72 that the user is sleeping (YES in step S72), it gives alarm (performs the notification operation) (step S74). Notification controller 278 plays a music file of sound A included in the setting information stored in memory 23 and controls speaker 22 to provide output of the music file. Then, the process proceeds to "C". Specifically, the alarm issuance stop processing in step ST8 in Fig. 10 is performed.

When CPU 27 determines in step S72 that the user is not sleeping (NO in step S72), it checks presence/absence (step S76). Specifically, presence/absence determination unit 271 makes determination as to presence/absence of the user based on a signal from Doppler sensor 30 and provides a result of determination to notification controller 278.

CPU 27 makes determination as to presence (step S78). Notification controller 278 makes determination as to presence based on a result of determination as to presence/absence from presence/absence determination unit 271.

When CPU 27 makes determination as presence in step S78 (YES in step S78), the process returns to step S70 and processing is continued.

On the other hand, when CPU 27 makes determination not as presence in step S78 (NO in step S78), the process proceeds to "D". In other words, the process proceeds to step S66 in Fig. 16. Notification controller 278 instructs speaker 22 to give voice guidance information indicative of quitting. For example, guidance "today's alarm ends" is given as voice guidance information indicative of quitting.

### <Reliable Alarm Function>

The reliable alarm function refers to a function to stop alarm (stop the notification operation) when the user leaving the bed (the prescribed region) is detected. Therefore, when the user is determined as not leaving the bed (the prescribed region), alarm (the notification operation) is not stopped. In the sense that the user can reliably be caused to get up because the user is stimulated to leave the bed (the prescribed region) for stopping issuance of alarm (the notification operation), the function is called the "reliable alarm function."

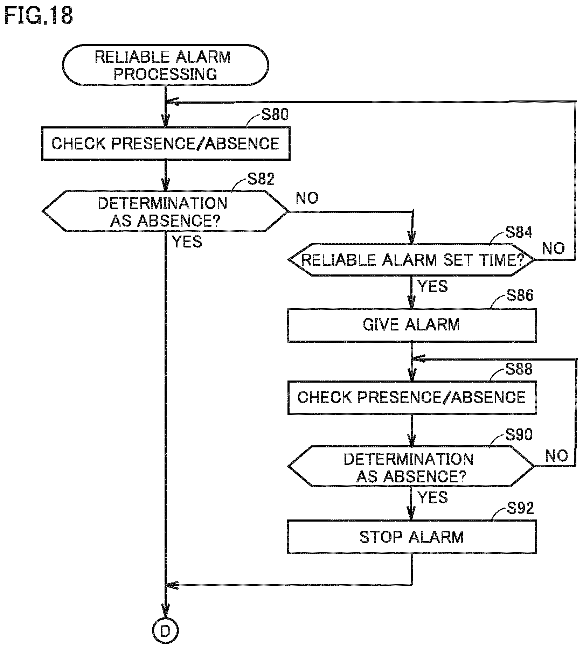

Fig. 18 is a diagram illustrating a flow of processing for the reliable alarm function based on the embodiment.

As shown in Fig. 18, CPU 27 checks presence/absence (step S80). Specifically, presence/absence determination unit 271 makes determination as to presence/absence of the user based on a signal from Doppler sensor 30 and provides a result of determination to notification controller 278.

CPU 27 makes determination as to absence (step S82). Notification controller 278 makes determination as to absence based on a result of determination as to presence/absence from presence/absence determination unit 271.

When CPU 27 makes determination as absence in step S82 (YES in step S82), the process proceeds to "D". In other words, the process proceeds to step S66 in Fig. 16. Notification controller 278 instructs speaker 22 to give voice guidance information indicative of quitting. For example, guidance "today's alarm ends" is given as voice guidance information indicative of quitting.

On the other hand, when CPU 27 makes determination not as absence in step S82 (NO in step S82), it determines whether or not reliable alarm set time has come (step S84). Notification controller 278 determines whether or not the reliable alarm set time included in the setting information has come.

When CPU 27 determines that the reliable alarm time has come, it gives alarm (performs the notification operation) (step S86). Notification controller 278 plays a music file of sound A included in the setting information stored in memory 23 and controls speaker 22 to provide output of the music file.

CPU 27 checks presence/absence (step S88). Specifically, presence/absence determination unit 271 makes determination as to presence/absence of the user based on a signal from Doppler sensor 30 and provides a result of determination to notification controller 278.

CPU 27 makes determination as to absence in step S90 (step S90). Notification controller 278 makes determination as to absence based on a result of determination as to presence/absence from presence/absence determination unit 271.

When CPU 27 makes determination as absence in step S90 (YES in step S90), it stops alarm (the notification operation) (step S92). Notification controller 278 stops output of alarm sound from speaker 22.

Then, the process proceeds to "D". In other words, the process proceeds to step S66 in Fig. 16. Notification controller 278 instructs speaker 22 to give voice guidance information indicative of quitting. For example, guidance "today's alarm ends" is given as voice guidance information indicative of quitting.

When CPU 27 makes determination not as absence in step S90 (NO in step S90), the process returns to step S88 and the processing above is repeated.

Therefore, when the user is determined as not leaving the bed (prescribed region), alarm (the notification operation) is not stopped. The user is stimulated to leave the bed (prescribed region) in order to stop alarm (the notification operation).

Though processing for adjusting the volume of output from speaker 22 is not described above, for example, possibility of absence as the result of determination by presence/absence determination unit 271 may be determined and the volume may be adjusted in accordance with the possibility of absence. For example, when the possibility of absence is high, the volume of output from speaker 22 may be lowered to half. Furthermore, when the possibility of absence subsequently becomes higher, alarm (the notification operation) may be stopped. As a result of the processing, for example, stop due to erroneous recognition in spite of the user still staying in bed can be prevented, and such a situation that the alarm (the notification operation) is not stopped with the volume remaining high in spite of the user having left the bed can also be prevented.

When the alarm function ends, CPU 27 performs processing for analyzing data on measured sleep of the user and performs processing for transmitting a result of analysis to server 6 and terminal 8 through communication apparatus 24. The user can thus perform a "view sleep" function which will be described later, with the use of terminal 8.

### <Light-Sleep Induction Function>

The light-sleep induction function refers to a function to induce the sleep state of the user from the deep sleep state to the light sleep state. The light-sleep induction function is processing performed in parallel to the processing for the alarm function described with reference to Fig. 10.

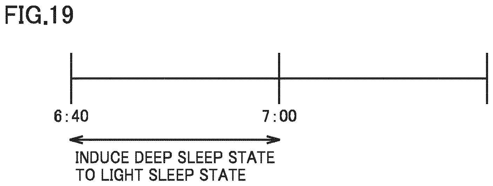

Fig. 19 is a conceptual diagram illustrating the light-sleep induction function.

As shown in Fig. 19, the light-sleep induction function is activated twenty minutes before get-up set time.

Specifically, the sleep state of the user is checked, and when the sleep state of the user falls under the deep sleep state, the user is induced from the deep sleep state to the light sleep state. When the sleep state of the user does not fall under the deep sleep state twenty minutes before get-up set time, the function is not activated.

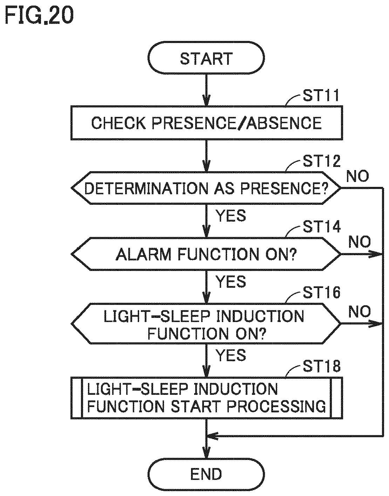

Fig. 20 is a diagram illustrating a flow of processing for the light-sleep induction function based on the embodiment.

As shown in Fig. 20, CPU 27 checks presence/absence (step ST11). Specifically, presence/absence determination unit 271 makes determination as to presence/absence based on a signal from Doppler sensor 30 and provides a result of determination to alarm function execution determining unit 280.

CPU 27 makes determination as to presence (step ST12). Alarm function execution determining unit 280 makes determination as to presence based on a result of determination as to presence/absence from presence/absence determination unit 271.

When CPU 27 makes determination as presence (YES in step ST12), it determines whether or not the alarm function is ON (step ST14). When determination as presence is made based on the result of determination as to presence/absence from presence/absence determination unit 271, alarm function execution determining unit 280 determines whether or not the setting information includes information that the alarm function is ON.

When CPU 27 determines in step ST14 that the alarm function is ON (YES in step ST14), it determines whether or not the light-sleep induction function is ON (step ST16). Alarm function execution determining unit 280 determines whether or not the setting information includes information that the light-sleep induction function is ON.

When CPU 27 determines in step ST16 that the light-sleep induction function is ON, it performs light-sleep induction function start processing (step ST18). Alarm function execution determining unit 280 instructs sleep state inducer 277 to perform the light-sleep induction function start processing. Details of the light-sleep induction function start processing will be described later.

Then, the process ends (end).

On the other hand, when CPU 27 makes determination not as presence in step ST12 (NO in step ST12), the process ends (end). Alarm function execution determining unit 280 does not instruct sleep state inducer 277 to perform the light-sleep induction function start processing.

When CPU 27 determines in step ST14 that the alarm function is not ON (NO in step ST14), the process ends (end). Alarm function execution determining unit 280 does not instruct sleep state inducer 277 to perform the light-sleep induction function start processing.

When CPU 27 determines in step ST16 that the light-sleep induction function is not ON (NO in step ST16), the process ends (end). Alarm function execution determining unit 280 does not instruct sleep state inducer 277 to perform the light-sleep induction function start processing.

Through the processing, when the user is not present on the bed which is the region of monitoring by sleep alarm apparatus 2, when the user desires to turn off the alarm function (the alarm function being OFF), or when the light-sleep induction function is OFF, the light-sleep induction function is not performed. Therefore, the function being performed uselessly can be suppressed and user's intention can be reflected.

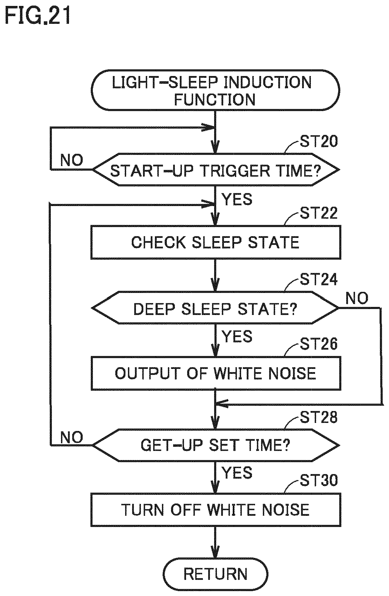

Fig. 21 is a diagram illustrating a flow of the light-sleep induction function start processing based on the embodiment.

As shown in Fig. 21, CPU 27 determines whether or not start-up trigger time has come (step ST20). The sleep state inducer determines whether or not the current time is time a prescribed period before (twenty minutes before by way of example) get-up set time.

CPU 27 maintains a state until the start-up trigger time comes (NO in step ST20).

On the other hand, when CPU 27 determines that the start-up trigger time comes (YES in step ST20), it checks the sleep state (step ST22). Sleep state determination unit 272 determines the sleep state of the user based on a signal from Doppler sensor 30 and provides a result of determination to sleep state inducer 277.

CPU 27 determines whether or not the sleep state of the user falls under the deep sleep state (step ST24). Sleep state inducer 277 determines whether or not the sleep state of the user falls under the deep sleep state based on a result of determination from sleep state determination unit 272.

When CPU 27 determines in step S24 that the sleep state of the user falls under the deep sleep state (YES in step ST24), it has output of white noise provided (step ST26). Sleep state inducer 277 has output of white noise provided through speaker 22.

Then, CPU 27 determines whether or not the get-up set time has come (step ST28). Sleep state inducer 277 determines whether or not the get-up set time included in the setting information has come.

When CPU 27 determines in step ST28 that the get-up set time has come (YES in step ST28), it turns off white noise (step ST30). When sleep state inducer 277 determines that the get-up set time has come, it stops output of white noise through speaker 22.

Then, the process ends (return).

On the other hand, when CPU 27 determines that the get-up set time has not yet come (NO in step ST28), the process returns to step ST22 and the processing above is repeated.

When CPU 27 determines in step ST24 that the sleep state of the user does not fall under the deep sleep state (NO in step ST24), step ST26 is skipped and the process proceeds to step ST28. Subsequent processing is similar.

When CPU 27 determines in step ST24 that the sleep state of the user does not fall under the deep sleep state (NO in step ST24) and when output of white noise has already been provided, it may stop the output. Then, when CPU 27 determines again that the sleep state of the user falls under the deep sleep state, it may have output of white noise provided again. Though an example in which output of white noise is stopped when the user is determined as not being in the deep sleep state is described in the present example, without being limited as such, for example, output of white noise may be stopped when body motion of the user such as turn-over is sensed.

Therefore, when the sleep state of the user a prescribed period before get-up set time is checked and the sleep state of the user falls under the deep sleep state, output of white noise is provided to induce the sleep state from the deep sleep state to the light sleep state. When the sleep state of the user does not fall under the deep sleep state the prescribed period before get-up set time, output of white noise is not provided.

Since the sleep state can be induced to the light sleep state before the get-up set time, the user can get up easily.

### <Morning Word Function>

The morning word function refers to a function to give information desired by the user at the timing desired by the user. The morning word function is processing performed in parallel to the processing for the alarm function described with reference to Fig. 10.

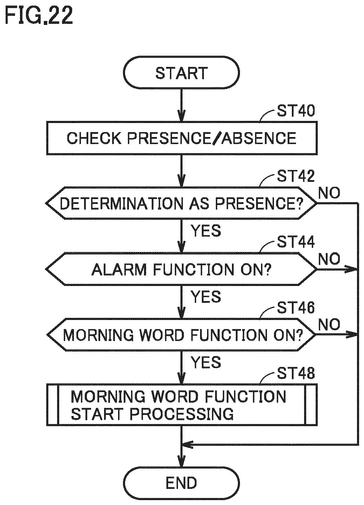

Fig. 22 is a diagram illustrating a flow of processing for the morning word function based on the embodiment.

As shown in Fig. 22, CPU 27 checks presence/absence (step ST40). Specifically, presence/absence determination unit 271 provides a result of determination as to presence/absence based on a signal from Doppler sensor 30 to alarm function execution determining unit 280.

CPU 27 makes determination as to presence (step ST42). Alarm function execution determining unit 280 makes determination as to presence based on a result of determination as to presence/absence from presence/absence determination unit 271.

When CPU 27 makes determination as presence (YES in step ST42), it determines whether or not the alarm function is ON (step ST44). When determination as presence is made based on a result of determination as to presence/absence from presence/absence determination unit 271, alarm function execution determining unit 280 determines whether or not the setting information includes information that the alarm function is ON.

When CPU 27 determines in step ST44 that the alarm function is ON (YES in step ST44), it determines whether or not the morning word function is ON (step ST46). Alarm function execution determining unit 280 determines whether or not the setting information includes information that the morning word function is ON.

When CPU 27 determines in step ST46 that the morning word function is ON, it performs morning word function start processing (step ST48). Alarm function execution determining unit 280 instructs comment issuing unit 279 to perform the morning word function start processing. Details of the morning word function start processing will be described later.

Then, the process ends (end).

On the other hand, when CPU 27 makes determination not as presence in step ST42 (NO in step ST42), the process ends (end). Alarm function execution determining unit 280 does not instruct comment issuing unit 279 to perform the morning word function start processing.

When CPU 27 determines in step ST44 that the alarm function is not ON (NO in step ST44), the process ends (end). Alarm function execution determining unit 280 does not instruct comment issuing unit 279 to perform the morning word function start processing.

When CPU 27 determines in step ST46 that the morning word function is not ON (NO in step ST46), the process ends (end). Alarm function execution determining unit 280 does not instruct comment issuing unit 279 to perform the morning word function start processing.

Through the processing, when the user is not present on the bed which is the region of monitoring by sleep alarm apparatus 2, when the user desires to turn off the alarm function (the alarm function being OFF), or when the morning word function is OFF, the morning word function is not performed. Therefore, the function being performed uselessly can be suppressed and user's intention can be reflected.

Though an example in which the morning word function is not performed when the alarm function is OFF is described above, the morning word function start processing may be performed when the morning word function is ON even though the alarm function is OFF. In other words, a scheme without processing in step ST44 is also applicable.

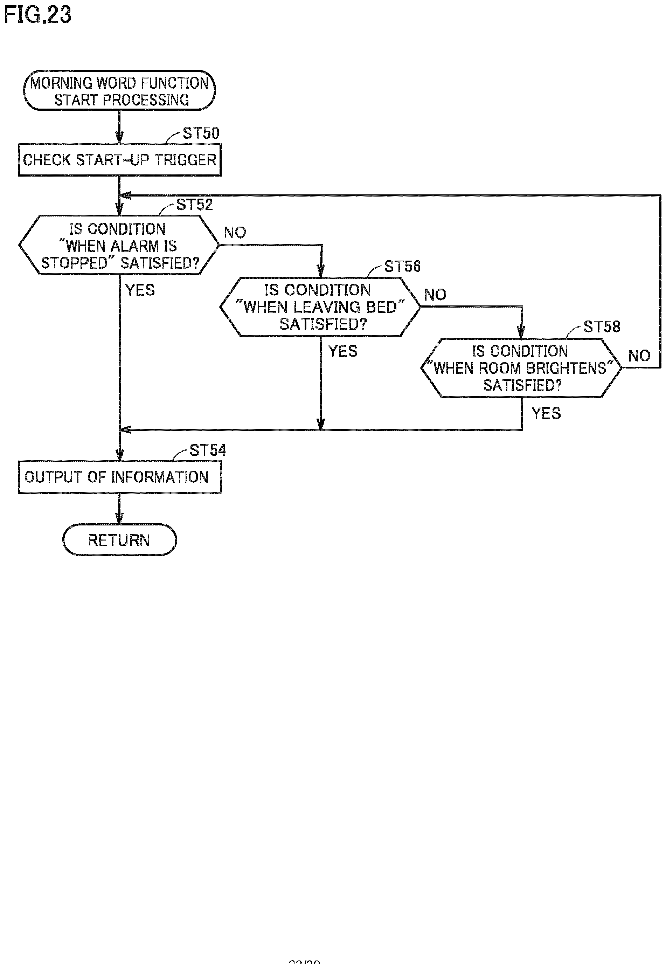

Fig. 23 is a diagram illustrating a flow of the morning word function start processing based on the embodiment.

As shown in Fig. 23, CPU 27 checks a start-up trigger (step ST50).

Then, CPU 27 determines whether or not the condition "when alarm is stopped" is satisfied (step ST52). Comment issuing unit 279 determines whether or not issuance of alarm (the notification operation) has been stopped when timing to perform the morning word function included in the setting information includes "when alarm is stopped."

When CPU 27 determines that the condition "when alarm is stopped" is satisfied (YES in step ST52), it provides information (step ST54). When the timing to perform the morning word function included in the setting information includes "when alarm is stopped" and when comment issuing unit 279 determines that issuance of alarm (the notification operation) has been stopped (the condition is satisfied), information on weather and a temperature on that day is provided as the information. Information obtained in advance and stored in memory 23 may be used as the information on the weather and the temperature, or the information may be obtained and used as a result of communication by comment issuing unit 279 with an external apparatus connected to network 4 through communication apparatus 24. Though an example in which information on the weather and the temperature is provided as the information is described by way of example, without being limited to the information on the weather and the temperature, any information convenient for the user such as latest news or traffic information may be applicable.

Then, the process ends (return).

When the condition for stopping the alarm is not satisfied in step ST52 (NO in step ST52), CPU 27 determines whether or not the condition "when leaving bed" is satisfied (step ST56). When timing to perform the morning word function included in the setting information includes "when leaving bed," comment issuing unit 279 determines whether or not switching from determination as presence to determination as absence of the user has been made based on a result of determination as to presence/absence from presence/absence determination unit 271.

When CPU 27 determines that the condition "when leaving bed" is satisfied (YES in step ST56), it provides information (step ST54). When timing to perform the morning word function included in the setting information includes "when leaving bed" and comment issuing unit 279 determines that switching from determination as presence to determination as absence of the user has been made (the condition is satisfied), comment issuing unit 279 provides information on the weather and the temperature as the information.

Then, the process ends (return).

When the condition "when leaving bed" is not satisfied in step ST56 (NO in step ST56), CPU 27 determines whether or not the condition "when room brightens" is satisfied (step ST58). When timing to perform the morning word function included in the setting information includes "when room brightens," comment issuing unit 279 determines whether or not the room has brightened based on an illuminance value from illuminance sensor 26.

When CPU 27 determines in step ST58 that the condition "when room brightens" has been satisfied (YES in step ST58), it provides information (step S54). When timing to perform the morning word function included in the setting information includes "when room brightens" and when comment issuing unit 279 determines that the room has brightened based on the illuminance value from illuminance sensor 26 (the condition is satisfied), comment issuing unit 279 provides information on the weather and the temperature as the information.

Then, the process ends (return).

When CPU 27 determines in step ST58 that the condition "when room brightens" has not been satisfied (NO in step ST58), the process returns to step ST52 and the processing above is repeated.

Therefore, when timing to perform the morning word function is checked and the condition is satisfied, information on the weather and the temperature is provided. The user can thus obtain necessary information at the timing desired by the user.

Though three types of "when alarm is stopped," "when leaving bed," and "when room brightens" are described as timing to perform the morning word function in the present example, without being limited as such, more types of timing may also be provided and set.

### <View Sleep>

The user can check information on sleep of the user based on sleep data stored in server 6 through terminal 8. For example, the user operates terminal 8 after the user wakes up. Sleep information based on sleep data stored in server 6 is shown on display 82 of terminal 8 in response to an operation instruction from the user.

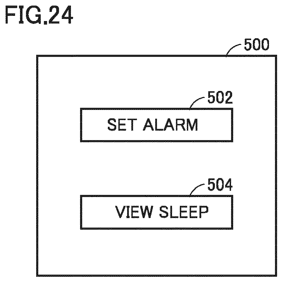

Fig. 24 is a diagram illustrating a top screen 500 of terminal 8 based on the embodiment.

As shown in Fig. 24, a plurality of buttons are provided in top screen 500.

Top screen 500 is provided with a "set alarm" button 502 and a "view sleep" button 504.

When the user selects "set alarm" button 502 on the touch panel, the alarm setting screen in Fig. 6 is shown. When the user selects "view sleep" button 504 on the touch panel, sleep information checking processing is performed. The sleep information checking processing is performed by execution by CPU 80 of a program stored in memory 86.

Contents at the time when "view sleep" button 504 is pressed are different between states before and after leaving the bed.

Specifically, before the user leaves the bed, the user may sleep in and data on the time when the user left the bed does not exist. Therefore, sleep data of the user has not been completed (finalized). It is inconvenient, however, that sleep data cannot be viewed unless the user leaves the bed. Therefore, before the user leaves the bed, data different from data at the time when the user leaves the bed may be shown.

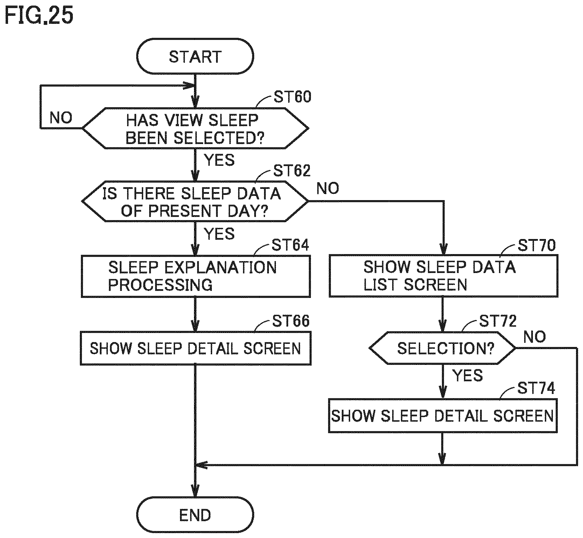

Fig. 25 is a diagram illustrating a flow of the sleep information checking processing in terminal 8 based on the embodiment.

As shown in Fig. 25, CPU 80 determines whether or not "view sleep" has been selected in top screen 500 (step ST60).

When "view sleep" is selected in top screen 50 in step ST60 (YES in step ST60), CPU 80 determines whether or not there is sleep data on the present day (step ST62). Specifically, CPU 80 accesses server 6 and determines whether or not there is sleep data on the present day transmitted from sleep alarm apparatus 2 to server 6.

In step ST60, CPU 80 maintains its state until "view sleep" is selected.

When CPU 80 determines that there is sleep data on the present day (YES in step ST62), it performs sleep explanation processing (step ST64). The sleep explanation processing refers to processing for giving contents resulting from analysis of sleep based on the sleep data on the present day. Sleep analysis is conducted by sleep alarm apparatus 2.

Characteristics and problems of sleep as a result of sleep analysis are given in a form of comments. Notification of a state of sleep debt may be given. When the state of sleep debt is not appropriate, a screen showing issuance of a warning to the effect that fatigue has been built up which poses a risk, together with comments including contents to advise the user to sleep, may be shown. Comments for improving sleep based on the characteristics and the problems of sleep may be given.

For example, characteristics of getting-up represented by body motion such as turn-over from thirty minutes before the user gets up and a result of how each function for wake-up worked (time of sounding and stop of alarm and change in volume of alarm) in accordance therewith may be presented in a graph.

Alternatively, a result of getting up and setting of the get-up mode may be referred to, and when the user got up at the get-up set time as set, that fact may be pointed out.

If a result of wake-up indicates that "deep sleep appears before getting up," "reaction to alarm is late," and "snooze is repeated at least five times" and a function to solve such problems has not been selected, recommendation of a corresponding function (for example, the "light-sleep induction function") may be shown. If a problem is observed in spite of use of the corresponding function, advice as to change in volume and alarm sound may be given at the end of the comments.

Without being limited to getting up, also for falling asleep, characteristics and problems in falling asleep may be given as comments.

Information on a falling-asleep function may be given. The falling-asleep function refers to a function for induction to the sleep state. For example, the falling-asleep function refers to a function to play music to relax the user to induce the user to the sleep state or to show a breathing method or relaxing exercise to induce the user to the sleep state.

In using the falling-asleep function, when the user is found to have successfully fallen asleep within a time period shorter than fifteen minutes after start of the function by referring to a result of falling asleep and function setting, the fact that the user has successfully fallen asleep with that function may be pointed out. When the result of falling asleep indicates that fifteen minutes or longer were spent before falling asleep and the falling-asleep function was not used, recommendation of the function to promote falling-asleep may be shown. If the falling-asleep function has already been used and fifteen or more minutes were spent to fall asleep, another unused falling-asleep function may be picked up and notification about the function may be given in a form of comments.

Then, CPU 80 has a sleep detail screen shown (step ST66).

Then, the process ends (end).

When CPU 80 determines in step ST62 that there is no sleep data on the present day (NO in step ST62), it has a sleep data list screen shown (step ST70).

Then, CPU 80 determines whether or not selection has been made (step ST72).

When CPU 80 determines in step ST72 that selection has been made (YES in step ST72), it has the sleep detail screen shown (step ST74).

When selection has not been made (NO in step ST72), CPU 80 skips step ST74 and quits the process (end).

Therefore, for example, when the user operates terminal 8 after the user wakes up, the sleep explanation processing in step ST64 is forcibly performed. Therefore, the user can see comments on the sleep state of the user owing to the sleep explanation processing. In other words, by properly knowing a condition of health of the user every day, health can readily be maintained and managed.

Though an example in which sleep explanation processing on the day when the user wakes up is performed is described in the present example, limitation thereto is not intended. For example, sleep explanation processing for a whole week may be performed at the end of a week, and comments on the sleep state for the week may be checked. Without being limited to one week, sleep explanation processing for a whole period may be performed at the end of one month or one year.

Alternatively, information on user's worries about sleep set in advance by providing inputs or making selection may be obtained and contents in comments in the sleep information checking processing may be changed based on the obtained information. For example, for a user who has worries about falling asleep, a result of analysis relating to falling asleep may conveniently be provided.

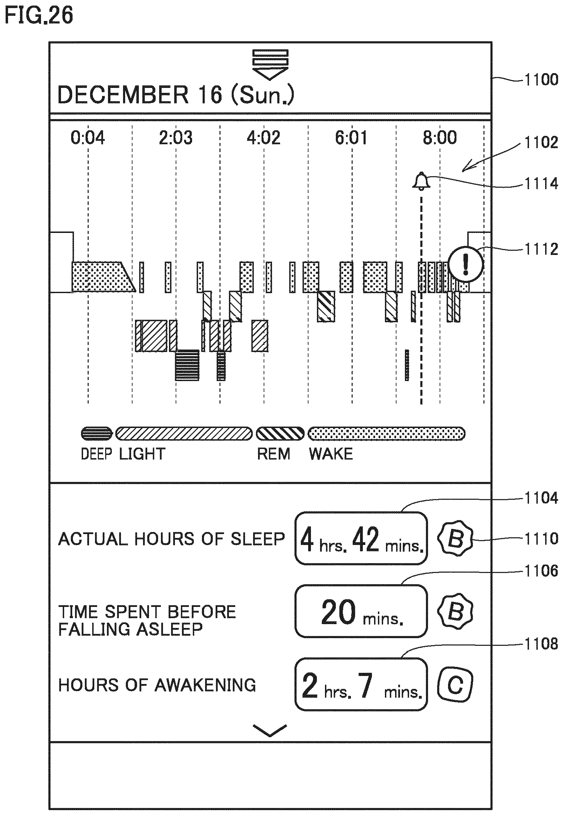

Fig. 26 is a diagram illustrating a sleep detail screen 1100 (No. 1) based on the embodiment.

As shown in Fig. 26, sleep detail screen 1100 is provided with a hypnogram (sleep diagram) 1102 and a plurality of sleep analysis items.

Hypnogram (sleep diagram) 1102 shows, with categorization into the deep sleep state, the light sleep state, a REM sleep state, and a wake state over the entire sleep period being made, a length of a period in each state.

In hypnogram (sleep diagram) 1102, a first graph (a hatched region in a top tier) shows the wake state. A second graph (a hatched region in a second tier) shows the REM sleep state. A third graph (a hatched region in a third tier) shows the light sleep state. A fourth graph (a hatched region in a fourth tier) shows the deep sleep state.

Hypnogram 1102 is provided with a bell object 1114 which shows get-up set time. In addition, an attention object 1112 is provided, and attention comments based on the sleep analysis item can be shown by selecting attention object 1112.

Therefore, with the hypnogram (sleep diagram), a distribution of each state can be known at a glance.

Though an example in which the sleep state of the user based on a detection signal from Doppler sensor 30 is analyzed and shown is described in the present example, ambient environmental data may be obtained in connection therewith and shown in association with the information on the sleep state. Specifically, information on brightness of the room provided from illuminance sensor 26 and audio information provided through microphone 28 may be obtained, and the information may be analyzed and shown. For example, a graph of a noise level and a brightness level around bed BD during sleep may be shown next to hypnogram 1102, together with the sleep state.

The audio information provided through microphone 28 includes environmental sound during sleep and sound emitted from the user (snore, somniloquy, and grinding), and sound emitted from the user may be extracted by analysis and reproduced. Time when sound was emitted from the user may also be shown in association with hypnogram 1102.

How many times and when the user has turned over may be shown in association with hypnogram 1102.

Specific examples of the sleep analysis items include "4 hrs. 42 mins." shown as an "actual hours of sleep" item 1104, "20 mins." shown as a "time spent before falling asleep" item 1106, and "2 hrs. 7 mins." shown as an "hours of awakening" item 1108.

An evaluation object 1110 is provided for each item. For example, by way of example, an evaluation object "B" is shown for the "actual hours of sleep" item. Similarly, an evaluation object "C" is shown for the "time spent before falling asleep" item. The evaluation object "C" is shown for the "hours of awakening" item. Based on evaluation indicated by the evaluation object, relative evaluation based on comparison with sleep data of an average general person can be known. Though an example in which relative evaluation objects from "A" to "C" are shown is described in the present example, without being particularly limited as such, a score value expressed in a numeric value may be shown.

When the "actual hours of sleep" are short and when the "actual hours of sleep" during a "time period from start of sleep until last wake-up" occupy less than ninety percent, an "undiscovered lack of sleep" object may be presented adjacent to the evaluation object.

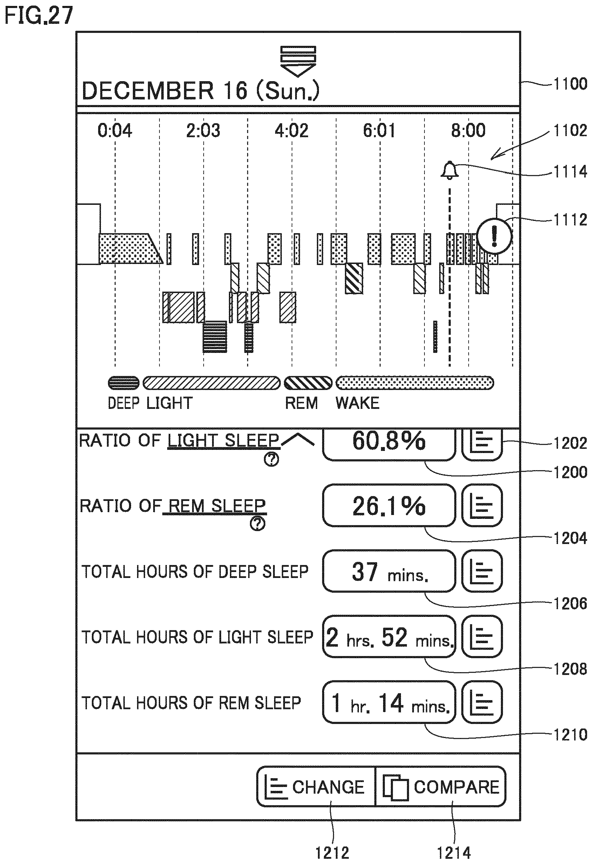

Fig. 27 is a diagram illustrating sleep detail screen 1100 (No. 2) based on the embodiment.

As shown in Fig. 27, sleep detail screen 1100 is provided with hypnogram (sleep diagram) 1102 and a plurality of sleep analysis items.

Specific examples of the sleep analysis items include "60.8%" shown for a "ratio of light sleep" item 1200, "26.1%" shown for a "ratio of REM sleep" item 1204, "37 mins." shown for a "total hours of deep sleep" item 1206, "2 hrs. 52 mins." shown for a "total hours of light sleep" item 1208, and " 1 hr. 14 mins." shown for a "total hours of REM sleep" item 1210.

A "change" button or a "compare" button is provided in correspondence with each item. Specifically, when a switch button 1212 to change to the "change button" is selected, the "change" button is provided in correspondence with each item. When a switch button 1214 to change to the "compare button" is selected, the "compare" button is provided in correspondence with each item.

When the "change" button is selected, a state of change in connection with the item for each date can be checked.

When the "compare" button is selected, comparison with average data for each age or sex can be made and a result can be checked. Sleep data of an animal or a fictitious character may be compared with sleep data of the user.

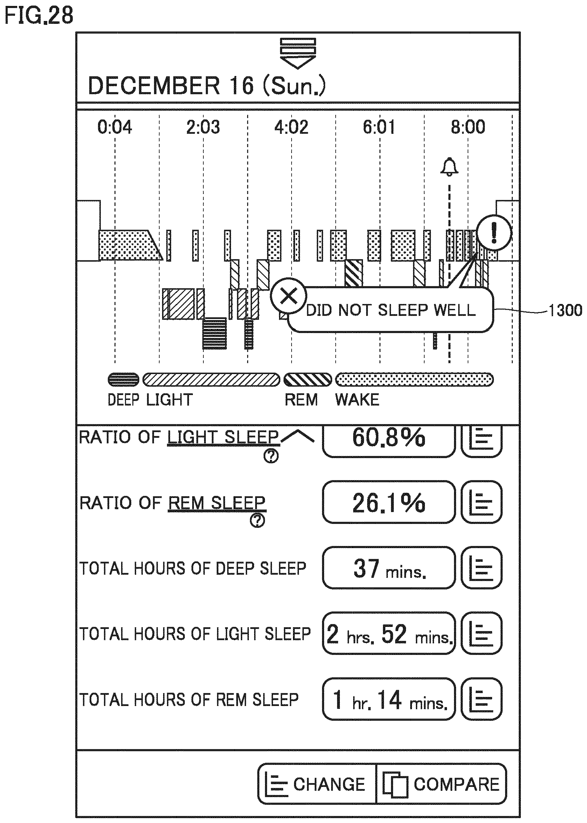

Fig. 28 is a diagram illustrating sleep detail screen 1100 (No. 3) based on the embodiment.

As shown in Fig. 28, sleep detail screen 1100 is provided with hypnogram (sleep diagram) 1102 and a plurality of sleep analysis items.

When attention object 1112 is selected, attention comments 1300 are shown. "Did not sleep well" is shown as exemplary attention comments.

With the attention comments, the sleep state of the user can readily be checked.

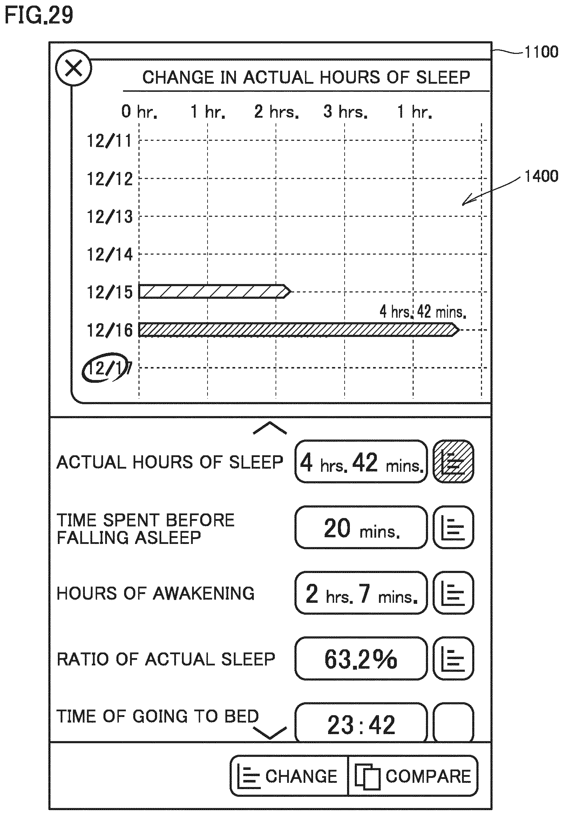

Fig. 29 is a diagram illustrating sleep detail screen 1100 (No. 4) based on the embodiment.

As shown in Fig. 29, sleep detail screen 1100 is provided with actual hours of sleep on each date and a plurality of sleep analysis items.

A graph at the time when the change button is selected for the "actual hours of sleep" item is shown.

By way of example, a graph 1400 shows actual hours of sleep on each date.

Change in actual hours of sleep for each date can thus readily be known.

Without being limited to daily change, weekly, monthly, or yearly change may be checked.

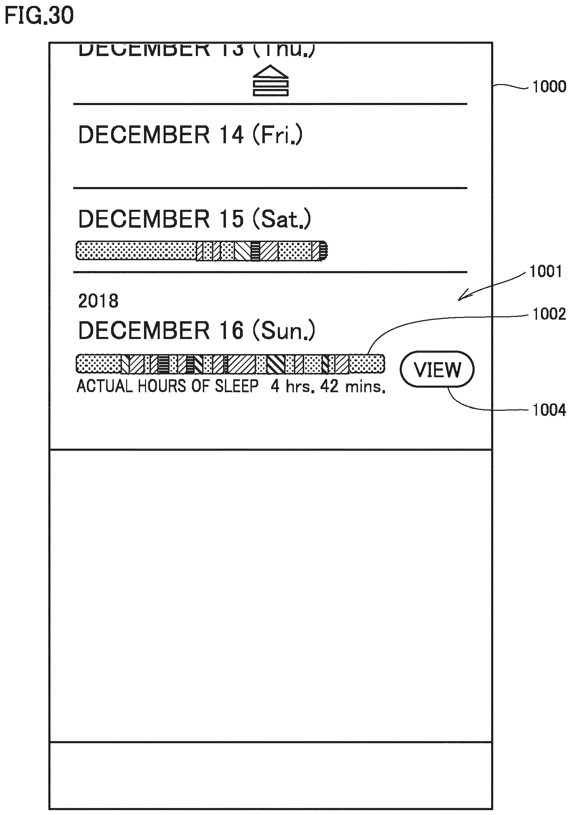

Fig. 30 is a diagram illustrating a sleep data list screen 1000 based on the embodiment.

As shown in Fig. 30, sleep data list screen 1000 shows a list of sleep data. A plurality of list items 1001 are provided.

Each user who uses the list can check a list of sleep data categorized for each date through the touch panel.

In the present example, an example in which sleep data on "December 16 (Sun), 2018" is selected is shown.

A sleep bar 1002 is shown within list item 1001. Sleep bar 1002 shows, with categorization of the sleep state (the deep sleep state, the light sleep state, the REM sleep state, and the wake state) of the user over the entire sleep period being made, a length of a period in each state. With sleep bar 1002, a ratio of each state during sleep of the user can be known at a glance.

Actual hours of sleep are shown within list item 1001. In the present example, "4 hrs. 42 mins." is shown. The user can thus readily know the hours of sleep.

A "view" button 1004 is provided within list item 1001.

As the user selects "view" button 1004, the sleep detail screen on the selected date described in detail with reference to Fig. 26 can be shown.

A function to edit sleep data stored in server 6 through terminal 8 may be provided, and data may be registered again for allowing combination, deletion, or edition of a result of sleep for analysis again.

Though an example in which sleep is analyzed in sleep alarm apparatus 2 is described by way of example in the present example, without being limited to sleep alarm apparatus 2, server 6 may conduct analysis. Without being limited to processing for analysis of sleep, various types of functional processing described above may similarly be performed by any of server 6 and terminal 8, without being limited to sleep alarm apparatus 2.

An application executable by a personal computer may be provided as a program in the present embodiment. The program according to the present embodiment may be incorporated as some functions of various applications executed on the personal computer.

It should be understood that the embodiment disclosed herein is illustrative and non-restrictive in every respect. The scope of the present invention is defined by the terms of the claims rather than the description above and is intended to include any modifications within the scope and meaning equivalent to the terms of the claims.

### REFERENCE SIGNS LIST

1 sleep management system; 2 sleep alarm apparatus; 4 network; 6 server; 8 terminal; 20 clock; 21, 82 display; 22 speaker; 23, 62, 86 memory; 24, 64, 84 communication apparatus; 26 illuminance sensor; 28 microphone; 29, 88 input apparatus; 30 Doppler sensor; 32, 66, 89 internal bus; 100 alarm setting screen; 270 turn-over sensing unit; 271 absence determination unit; 272 sleep state determination unit; 273 sleep score calculator; 274 body motion sensing unit; 275 representation controller; 276 accumulator; 277 sleep state inducer; 278 notification controller; 279 comment issuing unit; 280 alarm function execution determining unit; 282 information transmitter

## Claims

1. An information processing apparatus comprising:
a time counter that manages time;
a notification unit that performs a notification operation for waking a user up;
a sensing unit that wirelessly senses a signal in accordance with motion of the user; and
a controller that controls the notification unit to perform the notification operation when a notification condition is satisfied and controls the notification unit to stop the notification operation when a notification stop condition is satisfied based on a result of sensing by the sensing unit.

2. The information processing apparatus according to claim 1, wherein
the sensing unit includes a Doppler sensor,
the controller includes
a body motion sensing unit that senses body motion of the user based on an output from the Doppler sensor,
a sleep state estimator that estimates a state of sleep of the user based on the output from the Doppler sensor, and
a notification controller that controls the notification unit to perform the notification operation when the notification condition is satisfied and controls the notification unit to stop the notification operation when the notification stop condition is satisfied based on a result of sensing by the body motion sensing unit.

3. The information processing apparatus according to claim 2, wherein
the sleep state estimator estimates the state of sleep of the user by sensing a breath signal from the Doppler sensor.

4. The information processing apparatus according to claim 2 or 3, wherein
the body motion sensing unit provides a detection value indicating magnitude of body motion of the user,
the controller further includes an accumulator that calculates a cumulative value from the detection value, and
the notification controller controls the notification unit to stop the notification operation when the cumulative value reaches a threshold value at which the notification stop condition is satisfied.

5. The information processing apparatus according to claim 4, wherein
the accumulator calculates the cumulative value from detection values over a period of a predetermined length.

6. The information processing apparatus according to claim 5, wherein
the accumulator calculates the cumulative value from the detection values over a recent detection period.

7. The information processing apparatus according to any one of claims 4 to 6, wherein
the controller further includes a representation controller that controls a display to show information indicating the cumulative value.

8. The information processing apparatus according to claim 7, wherein
the representation controller controls the display to show an indicator varied in length in accordance with magnitude of the cumulative value toward a position indicating the threshold value.

9. The information processing apparatus according to claim 2, wherein when a result of sensing by the body motion sensing unit indicates continued body motion of the user for a predetermined period, the notification controller controls the notification unit to stop the notification operation.

10. The information processing apparatus according to claim 2, wherein when the result of sensing by the body motion sensing unit does not indicate detection of body motion of the user while the state of sleep of the user estimated by the sleep state estimator falls under wake, the notification controller controls the notification unit not to stop the notification operation.

11. An information processing method comprising:
managing time;
performing a notification operation for waking a user up when a notification condition is satisfied;
wirelessly sensing a signal in accordance with motion of the user; and
stopping the notification operation when a notification stop condition is satisfied based on a result of sensing.

12. An information processing program executed by a computer including a notification unit that performs a notification operation for waking a user up and a sensing unit that wirelessly senses a signal in accordance with motion of the user, the information processing program causing the computer to perform:
managing time;
controlling the notification unit to perform the notification operation when a notification condition is satisfied; and
controlling the notification unit to stop the notification operation when a notification stop condition is satisfied based on a result of sensing by the sensing unit.

13. An information processing system comprising:
a time counter that manages time;
a notification unit that performs a notification operation for waking a user up;
a sensing unit that wirelessly senses a signal in accordance with motion of the user; and
a controller that controls the notification unit to perform the notification operation when a notification condition is satisfied and controls the notification unit to stop the notification operation when a notification stop condition is satisfied based on a result of sensing by the sensing unit.
